(19)

Europäisches Patentamt

European Patent Office

Office européen des brevets

(11) **EP 4 317 972 A2**

(12) **EUROPEAN PATENT APPLICATION**

(43) Date of publication:
**07.02.2024 Bulletin 2024/06**

(21) Application number: **23201626.1**

(22) Date of filing: **06.02.2019**

(51) International Patent Classification (IPC):
***G01N 33/50*** *(2006.01)*

(52) Cooperative Patent Classification (CPC):
**A61K 45/06; A61K 31/505; A61K 31/513;
A61K 31/675; A61K 31/706; A61K 31/7105;
A61K 31/713; C12Q 1/6886;** C12Q 2600/106;
C12Q 2600/158; G01N 2800/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2018 US 201862627151 P**

(62) Document number(s) of the earlier application(s) in accordance with Art. 76 EPC:
**19751681.8 / 3 749 330**

(71) Applicants:
• **The General Hospital Corporation
Boston, MA 02114 (US)**
• **Icahn School of Medicine at Mount Sinai
New York, NY 10029 (US)**

(72) Inventors:
• **TING, David T.
Dover (US)**
• **ARORA, Kshitij
North Quincy (US)**
• **RIVERA, Miguel N.
Belmont (US)**
• **DESHPANDE, Vikram
Belmont (US)**
• **GREENBAUM, Benjamin Dylan
New York (US)**
• **SOLOVYOV, Alexander V.
New York (US)**

(74) Representative: **Forresters IP LLP
Skygarden
Erika-Mann-Straße 11
80636 München (DE)**

Remarks:
•This application was filed on 04-10-2023 as a divisional application to the application mentioned under INID code 62.
•Claims filed after the date of filing of the application (Rule 68(4) EPC).

(54) **REPEAT RNA AS BIOMARKERS OF TUMOR IMMUNE RESPONSE**

(57) Methods for predicting response to immunotherapy and selecting immunotherapy for treating cancer, e.g., cancer of epithelial origin, in a subject.

EP 4 317 972 A2

*FIG. 7*

## Description

## CLAIM OF PRIORITY

This application claims the benefit of U.S. Provisional Patent Application Serial No. 62/627,151, filed on February 6, 2018. The entire contents of the foregoing are hereby incorporated by reference.

## FEDERALLY SPONSORED RESEARCH OR DEVELOPMENT

This invention was made with Government support under Grant No. CA087497 awarded by the National Institutes of Health and Grant No. 1545935 awarded by the National Science Foundation. The Government has certain rights in the invention.

## TECHNICAL FIELD

The present invention relates to methods for predicting response to immunotherapy and selecting immunotherapy for treating cancer, e.g., cancer of epithelial origin, in a subject.

## BACKGROUND

The human genome has a high percentage of non-protein coding genomic regions organized as tandemly repeated sequences. These genomic regions are normally transcriptionally silent, but can be transcribed in cancer cells. For example, the pericentromeric human satellite II (HSATII) sequence has been shown to be overexpressed in epithelial cancers (e.g., pancreatic cancer) yet silenced in normal cells (see Prosser et al. J. Mol. Biol. 187(2): 145-55 (1986); Warburton et al. (2008) BMC Genomics 9: 533; and Ting et al. (2011) Science 331(6017): 593-6; International Publication No. WO 2012/048113l).

## SUMMARY

Growing evidence indicates that innate immune pathway activation is critical for responses to immunotherapy and overall cancer prognosis. It has been posited that innate immunity in the tumor microenvironment can be driven by derepression of endogenous repetitive element RNA. The ability to characterize these species provides new predictive biomarkers for tumor immune responses and a mechanistic basis for elements of innate activation by tumors.

Thus, provided herein are methods for treating a subject with cancer in a subject. The methods include providing a sample comprising cells from the cancer; detecting a level of repeat RNA (e.g., HSATII, LINE-1, HERV-K, HERV-H) in the cells; comparing the level of repeat RNA in the sample to a reference level; and selecting and optionally administering a treatment comprising one or both of (i) a treatment that reduces levels of export of repeat RNA or increases levels of repeat RNA in the cells, or (ii) a tumor-protective immunosuppression reducing immunotherapy, to a subject who has a cancer that has levels of repeat RNA above a reference level. In some embodiments, the repeat RNA is HSATII.

In some embodiments, the treatment that reduces levels of export of repeat RNA or increases levels of repeat RNA in the cells is an inhibitory nucleic acid targeting repeat RNA, preferably selected from the group consisting of a locked nucleic acid (LNA) molecule, a short hairpin RNA (shRNA) molecule, a small inhibitory RNA (siRNA) molecule, an antisense nucleic acid molecule, a peptide nucleic acid molecule, and a morpholino.

In some embodiments, the treatment that levels of export of repeat RNA or increases levels of repeat RNA in the cells is a reverse transcriptase inhibitor (RTI), preferably selected from the group consisting of a nucleoside analog reverse transcriptase inhibitor, a nucleotide analog reverse transcriptase inhibitor, non-nucleoside reverse transcriptase inhibitor, and a combination thereof. In some embodiments, the nucleoside analog reverse transcriptase inhibitor comprises lamivudine, abacavir, zidovudine, emtricitabine, didanosine, stavudine, entecavir, apricitabine, censavudine, zalcitabine, dexelvucitabine, amdoxovir, elvucitabine, festinavir, racivir, stampidine, or a combination thereof. In some embodiments, the non-nucleoside reverse transcriptase inhibitor comprises lersivirine, rilpivirine, efavirenz, etravirine, doravirine, dapivirine, or a combination thereof; or wherein the nucleotide analog reverse transcriptase inhibitor comprises tenofovir alafenamide fumarate, tenofovir disoproxil fumarate, adefovir, or a combination thereof.

In some embodiments, the treatment that reduces levels of export of repeat RNA or increases levels of repeat RNA in the cells comprises a cytidine analog or a guanosine analog.

In some embodiments, the treatment that reduces levels of export of repeat RNA or increases levels of repeat RNA in the cells comprises an HDAC inhibitor; a BET inhibitor; or a DNA hypomethylating agent. In some embodiments, the DNA hypomethylating agent is azacytidine, decitabine, cladribine, or a combination thereof; the HDAC inhibitor is Suberoylanilide hydroxamic acid (SAHA/Vorinostat/Zolinza), Trichostatin A (TSA), PXD-101, depsipeptide, MS-275, MGCD0103, valproic acid, or Sodium phenylbutyrate, or the BET inhibitor is (+)-JQ1, I-BET762, OTX015, I-BET151, CPI203, PFI-1, MS436, CPI-0610, RVX2135, FT-1101, BAY1238097, INCB054329, TEN-010, GSK2820151, ZEN003694, BAY-299, BMS-986158, ABBV-075, GS-5829, or PLX51107.

In some embodiments, the methods include administering the treatment comprising one or both of (i) a treatment that reduces levels of export of repeat RNA or increases levels of repeat RNA in the cells or (ii) a tumor-protective immunosuppression reducing immunotherapy, for a time sufficient to reduce levels of repeat below a subsequent reference level, and then administering a treatment comprising an anti-tumor immunity enhancing immunotherapy to the subject, wherein the initial and subsequent reference levels can be the same or different.

In some embodiments, the anti-tumor immunity enhancing immunotherapy comprises an immunotherapy agent selected from the group consisting of an anti-PD-1 antibody, an anti-PD-L1 antibody, an anti-CD137 antibody, an anti-CTLA4 antibody, an anti-CD40 antibody, an anti-II,10 antibody, an anti-TGF-β antibody, and an anti-IL-6 antibody.

In some embodiments, administering the treatment results in a reduction in tumor burden in the subject.

In some embodiments, the cancer is an epithelial cancer, e.g., melanoma, pancreatic cancer, colorectal cancer, breast cancer, prostate cancer, renal cancer, ovarian cancer, lung cancer, pancreatic cancer, liver cancer, or urothelial cancer.

In some embodiments, the cancer is pancreatic ductal adenocarcinoma, intrahepatic cholangiocarcinoma, or hepatocellular carcinoma

In some embodiments, the cancer comprises a mutation in tumor protein p53 (TP53). The method can optionally include detecting the presence of a mutation in TP53, and selecting the subject on the basis of the presence of the mutation.

Also provided herein are methods for predicting whether a cancer will respond to a treatment comprising an anti-tumor immunity enhancing immunotherapy. The methods include providing a sample comprising cells from the cancer; detecting a level of repeat RNA in the cells; and comparing the level of repeat RNA in the sample to a reference level; wherein a cancer that has levels of repeat RNA above or below the reference level is likely to respond to a treatment comprising an anti-tumor immunity enhancing immunotherapy. In some embodiments, the repeat RNA is HSATII, and the presence of levels of HSATII RNA below the reference level indicates the presence of CD8+ T cells and that the cancer is likely to respond to a treatment comprising an anti-tumor immunity enhancing immunotherapy; in some embodiments, the repeat RNA is HERV-H, and the presence of levels of HERV-H RNA above the reference level indicates the absence of FOXP3 cells and that the cancer is likely to respond to a treatment comprising an anti-tumor immunity enhancing immunotherapy. In some embodiments, both levels of HSATII and HERV-H are determined, and a treatment comprising an anti-tumor immunity enhancing immunotherapy is selected and optionally administered to a subject who has a cancer that has HSATII levels at or below a threshold, and HERV-H levels above a threshold.

In addition, provided herein are methods for predicting whether a cancer will respond to a treatment comprising a tumor-protective immunosuppression reducing immunotherapy. The methods include providing a sample comprising cells from the cancer; detecting a level of repeat RNA in the cells; and comparing the level of repeat RNA in the sample to a reference level; wherein a cancer that has levels of repeat RNA above the reference level is likely to respond to a treatment comprising a tumor-protective immunosuppression reducing immunotherapy, an inhibitory nucleic acid targeting repeat RNA, or a reverse transcriptase inhibitor (RTI) selected from the group consisting of a nucleoside analog reverse transcriptase inhibitor, a nucleotide analog reverse transcriptase inhibitor, non-nucleoside reverse transcriptase inhibitor, and a combination thereof.

Further, provided herein are methods for determining a presence or level of immune cells in a cancer. The methods include providing a sample comprising cells from the cancer; detecting a level of repeat RNA, e.g., HSATII and/or HERV-H repeat RNA, in the cells; and comparing the level of repeat RNA, e.g., HSATII and/or HERV-H repeat RNA, in the sample to a reference level (e.g., a level in a normal adjacent cell or tissue); wherein: a cancer that has levels of HSATII repeat RNA above the reference level has CD8 T-cells in the cancer; a cancer that has levels of HSATII repeat RNA at or below the reference level has CD 163 macrophages; and a cancer that has levels of HERV-H repeat RNA above the reference level has FOXP3 T cells.

In some embodiments, the methods include categorizing the cancer as CD8+, CD163+, or FOXP3+ based on the level of HSATII and/or HERV-H in the cells.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

## DESCRIPTION OF DRAWINGS

The patent or application file contains at least one drawing executed in color. Copies of this patent or patent application publication with color drawing(s) will be provided by the Office upon request and payment of the necessary fee.

Figures 1A and Figure 1B: Representative image of colon tumor expressing low HERV-H, stained for HERV-H (in situ hybridization) and FOXP3+ Treg cells (immunohistochemistry) (Figure 1A) and those expressing high levels of HERV-H, stained for HERV-H (in situ hybridization) and FOXP3+ Treg cells (immunohistochemistry) (Figure 1B). All images taken at 400x magnification.

Figure 2: Quantitation of intratumoral FOXP3+ Treg cells in HERV-H LOW vs HIGH tumors. Error bars = SD.

Figures 3A and 3B: Representative images of colon tumor stained for CD8 protein expression (immunohistochemistry) and HSATII RNA (in situ hybridization). Figure 3A: low HSATII expression correlates with high CD8+ T cells infiltration. Figure 3B: high HSATII expression correlates with low CD8+ T cells infiltration.

Figure 4: Associated quantification of colon cancer intratumoral CD8+ T cell per field of view ($400 \times 200\mu m$). Tumor samples were classified as HSATII high or low expression following in situ hybridization staining. p-value=0.0004 (unpaired t-test).

Figures 5A and 5B: Representative images of melanoma anti-CTLA4 (ipilimumab) responder with low HSATII and high CD3 T-cells (Figure 5A) and anti-PD1 (nivolumab) non-responder with high HSATII and low CD3 T-cells (Figure 5B).

Figure 6: Representative images of colon tumor stained for CD163 protein expression (immunohistochemistry) and HSATII RNA (in situ hybridization) showing positive correlation of CD163 macrophages with HSATII RNA.

Figure 7: Associated quantification of colon cancer intratumoral CD163+ macrophages per field of view ($400 \times 200\mu m$). Tumor samples were classified as HSATII high or low expression following in situ hybridization staining. p-value<0.0001.

Figure 8: Representative image of Pancreatic Ductal Adenocarcinoma (PDAC) tumors stained for HSATII RNA (in-situ hybridization) and CD 163+ macrophages (immunohistochemistry).

Figure 9: Representative images of Pancreatic Ductal Adenocarcinoma (PDAC) tumors stained for HSATII (in-situ hybridization) and CD8+ T-cells (immunohistochemistry). Shown are HSATII negative (upper left panel), low (upper right panel), moderate (lower left panel), and high expression (lower right panel). Note the decrease in CD8 T-cells as HSATII expression increases.

Figure 10: Associated quantification of PDAC intratumoral CD8+ T-cells per field of view ($400 \times 200\mu m$). Tumor samples were classified as HSATII High or Low expression following in situ hybridization staining (p-value < 0.0001, unpaired t-test).

Figure 11: Representative images of Intrahepatic cholangiocarcinoma (ICC) tumors stained for HSATII (in situ hybridization) and CD8+ T-cells (immunohistochemistry). Shown are tumor samples that were HSATII negative (upper left), low (upper right), moderate (lower left), and high expression (lower right). Note the decrease in CD8 T-cells as HSATII expression increases.

Figure 12: Representative images of Hepatocellular carcinoma (HCC) tumors stained for HSATII and CD8+ T-cells CD8+ T-cell. Shown are tumor samples that were HSATII negative (upper left), low (upper right), and moderate (lower left). There were no HCC tumor samples that expressed high HSATII expression. Note the decrease in CD8+ T cells as HSATII expression increases.

Figure 13: Associated quantification of ICC intratumoral CD8+ T-cells per field of view ($400 \times 200\mu m$). Tumor samples were classified as HSATII High or Low expression following in situ hybridization staininig (p-value = 0.0223, unpaired t-test).

Figure 14A: Heatmap for interferon-stimulated (viral defense) gene expression in urothelial carcinoma dataset from Snyder et al (PLoS Med. 14, e1002309).

Figure 14B: Kaplan-Meier plot for the overall survival between the patients from the ERV repeat high and ERV repeat low clusters. Association is significant (p=0.012, log rank test).

Figure 14C: Kaplan-Meier plot for the progression free survival between the patients from the ERV repeat high and ERV repeat low clusters. Association is significant (p=0.025, log rank test).

## DETAILED DESCRIPTION

Recent data has demonstrated that repeat RNA expression may drive the innate immune response in a wide array of malignancies including melanoma and cancers of the colon, pancreas, breast, liver, and lung (Rooney et al., Cell 160, 48-61 (2015); Chiappinelli et al., Cell 162, 974-986 (2015); Roulois et al., Cell 162, 961-973 (2015); Leonova et al., Proc Natl Acad Sci U S A 110, E89-98 (2013); Tanne et al., Proc Natl Acad Sci U S A 112, 15154-15159 (2015)).

Described herein are clinical grade RNA-ISH assays as well as methods for quantitating repeat RNAs by RNA-seq in cancer (See Appendix 1; Desai et al., JCI Insight 2, e91078 (2017); and Ting et al., Science 331, 593-596 (2011)). In addition, computational RNA-seq analysis in cancer genomics datasets (TCGA) indicated that many repeat RNAs are not currently captured by standard mRNA-seq datasets. The correlation of repeat RNAs in Total RNA-seq data was also seen (see Appendix 2, and Solovyov et al., bioRxiv, BIORXIV/2017/145946 (2017).

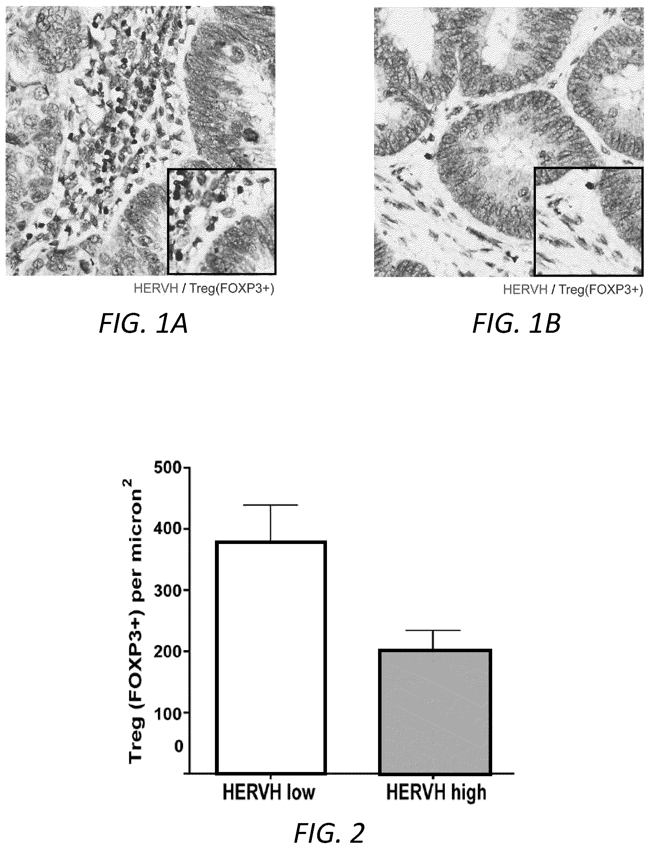

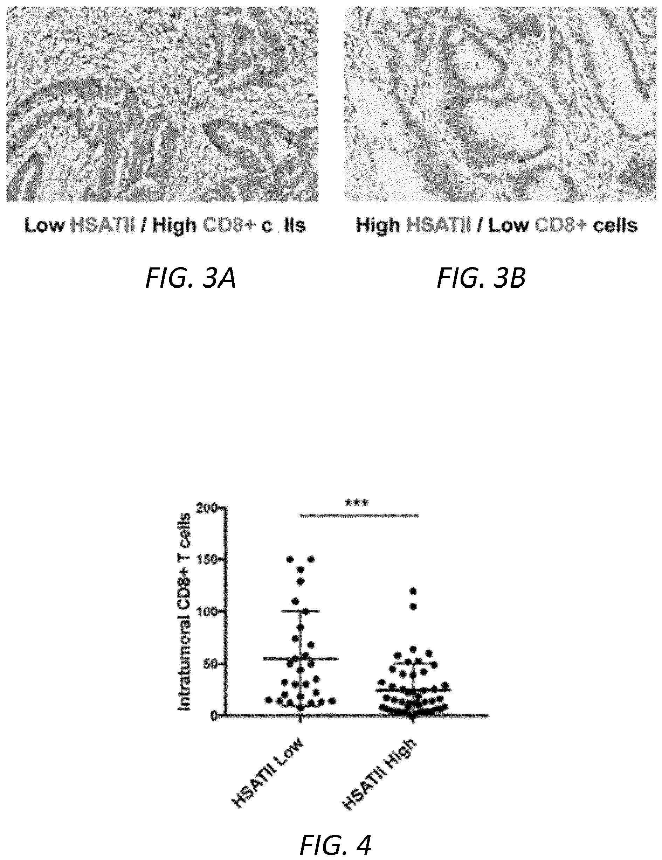

Described herein are assays that can be used to evaluate immune infiltrates (leukocytes including T-cell subsets (CD3, CD4, CD8, FOXP3); macrophage subsets (CD68, CD163); B-cells (CD20); NK subsets (CD56, CD16)) by immunohistochemistry (IHC) at the same time as the repeat RNA (HSATII, LINE1, HERV-H, and HERV-K) by RNA in situ hybridization (RNA-ISH). We have demonstrated in a cohort of 112 colon cancers an anti-correlation of HERV-H expression and FOXP3+ regulatory T cells (Figure 1 & 2) (see Appendix 1) and anti-correlation of HSATII expression and CD8+ effector T-cells (Figure 3&4) (see also Appendix 2).

The immunotherapy field has seen that CD8+ Tcell infiltrates in general correlate to response to immunotherapy (CTLA4 or PD1/PDL1). FOXP3+ regulatory T cells are thought to block the anti-tumor T cell response though this data has been more conflicted (Manjili MH and Butler SE Immunological Investigators 2016; Ward-Hartstonge KA and Kemp RA. Clinical & Translational Immunology 2017).

We predicted that HSATII low tumors correlate to HIGH CD8 T cell infiltrates and HERV-H high tumors correlate to LOW FOXP3+ cells, which would be most responsive to immunotherapy. As shown herein, HSATII low cancers, including melanomas, respond to immunotherapy including anti-PD1/PDL1 and anti-CTLA4 therapies. In addition, an HSATII high signal in cancer is positively correlated with CD163+ tumor associated macrophages, which is typically associated with an immunosuppressive or non-responsive tumor microenvironment.

Altogether, these data indicate that repeat RNA expression in tumor cells can be used, e.g., in circulating tumor cells (CTCs), exosomes (e.g. extracellular vesicles), fine needle aspirate or microcore biopsies to provide an assessment of the tumor immune microenvironment. These small biopsies are often too small to evaluate immune cell infiltrate status and cannot determine if a tumor is immune "hot" or "cold". Therefore, these repeat RNA immune markers are a surrogate of the tumor immune microenvironment. This has significant implications for cancer given that the vast majority of patients receiving immunotherapy drugs are metastatic cancer patients where only small biopsies are available.

As used herein, the term "cancer" refers to pathologic disease states, e.g., characterized by malignant tumor growth. In some embodiments, the methods described herein result in the delay or inhibition of tumor cell proliferation in a subject. In some embodiments, the methods described herein result in increased tumor cell death or killing in the subject. In some embodiments, the methods described herein result in the inhibition of a rate of tumor cell growth or metastasis. In some embodiments, the methods described herein result in a reduction in the size of a tumor in a subject. In some embodiments, the methods described herein result a reduction in tumor burden in a subject. In some embodiments, the methods described herein result in a reduction in the number of metastases in a subject.

As used herein, "prophylactic treatment" means reducing the incidence of or preventing (i.e., reducing risk of) a sign or symptom of a disease in a subject at risk for the disease, and "therapeutic treatment", which means reducing signs or symptoms of a disease, reducing progression of a disease, reducing severity of a disease, in a subject diagnosed with the disease.

The presence of cancer, e.g., solid tumors of epithelial origin, e.g., as defined by the ICD-O (International Classification of Diseases - Oncology) code (revision 3), section (8010-8790), e.g., early stage cancer, is associated with the presence of a massive levels of satellite due to increased transcription and processing of satellite repeats in epithelial cancer cells (see, e.g., Ting et al. (2011) Science 331(6017): 593-6; Bersani et al. (2015) Proc. Natl. Acad. Sci. U.S.A. 112(49): 15148-53; and U.S. Publication No. 2017/0198288 A1, the entire contents of each of which are expressly incorporated herein by reference).

**Using repeat RNA levels to predict response to and select therapy**

Applicants have identified a correlation between levels of repeat RNA, e.g., HSATII, LINE-1, HERV-K, HERV-H RNA, in cancer cells and the presence of specific populations of immune cells. As described herein, cancer cells having high levels of HSATII RNA are positively correlated with the presence of CD163+ tumor associated macrophages. Without wishing to be bound by theory, it is believed that the high levels of HSATII RNA in the tumors is associated with export of the HSATII RNA (e.g., by exosomes) resulting in high levels of HSATII RNA (e.g., in exosomes) present in the tumor environment, which attracts macrophages that then exert a protective effect, dampening the anti-tumor immune response. These cancers would be expected to respond to therapies that target the macrophages, or therapies that reduce levels of HSATII RNA transfer by exosomes, resulting in an increase in HSATII in the cell and cell death by necroptosis. This then decreases the amount of HSATII RNA exported to the macrophages to affect them. Thus, the methods described herein can include administering a macrophage-targeting therapy to a subject identified as having a cancer with high levels of HSATII RNA (i.e., levels of HSATII RNA above a selected threshold). In some embodiments, the methods can include administering a treatment that reduces levels of HSATII RNA export/increases levels of HSATII RNA in the cell to the subject, e.g., one or more HSATII Inhibitory Nucleic Acids; Reverse Transcription Inhibitors; a DNA hypomethylating agent such as a DNMT inhibitor; Histone deacetylase (HDAC) inhibitors; or Bromodomain and Extra-Terminal motif (BET) inhibitors; or a treatment that targets the macrophages, e.g., CD11b, CSF-1R, CCL2, Neuropilin-1, ANG2, IL-4, IL-4Ralpha, IL-13, FcgammaR, IL-6, IL-6R, TNF-alpha, CD40. Any of these treatments can be administered alone, together, or concurrently with or before administration of each other, or another anti-cancer therapy, e.g., cytotoxic chemotherapy. In some embodiments, after a subject is identified as having a cancer that has high levels of HSATII RNA (e.g., using an assay as described herein), a treatment that reduces levels of HSATII RNA exported from cancer cells and/or a treatment that reduces levels or modulates activity of macrophages is administered for a time sufficient to alter levels of HSATII RNA (e.g., to reduce levels of HSATII RNA export/increase levels of HSATII RNA in the cell) and/or increase levels of macrophages, e.g., for at least 2 days, 5 days, 7 days, 10 days, 14 days, 18 days, 21 days, 28 days, 30 days or more. Optionally, levels of HSATII RNA and/or levels of macrophages are determined again. Once the levels of HSATII in the cells have been increased, levels of HSATII in the exosomes in the tumor environment have been reduced, or the level of macrophages have been reduced (i.e., above or below a threshold), the methods can include administering a treatment as described herein for a subject who has an HSATII low cancer (i.e., a cancer with HSATII levels below a threshold).

HSATII low tumors correlate to the presence of high levels of CD8+ T cell infiltrates and cells having high levels of HERV-H are correlated with the absence of or low levels of Foxp3+ regulatory T cells; thus, the methods described herein can include administering a CD8+-targeting therapy to a subject identified as having a cancer with low levels of HSATII RNA and/or high levels of HERV-H, e.g., an immunotherapy that enhances the anti-tumor response.

In some embodiments, the methods described herein are used in treating a subject who has a cancer of epithelial origin (i.e., an epithelial cancer). Cancers of epithelial origin can include pancreatic cancer (e.g., pancreatic adenocarcinoma), lung cancer (e.g., non-small cell lung carcinoma or small cell lung carcinoma), prostate cancer, breast cancer, renal cancer, ovarian cancer, melanoma, or colon cancer. Satellites have also been shown to be elevated in preneoplastic or early cancer lesions including intraductal papillary mucinous neoplasm (IPMN), pancreatic intraepithelial neoplasia (PanIN), ductal carcinoma in situ (DCIS), Barrett's Esophagus (see e.g., Sharma (2009) N. Engl. J. Med. 361(26): 2548-56; erratum in: N Engl J Med. 362(15): 1450). Thus, the methods can be used to potentially treat early preneoplastic cancers as a means to prevent the development of invasive cancer. In some embodiments, the cancer is a microsatellite instable cancer (e.g., MSI colorectal cancer). In some embodiments, the cancer is a microsatellite stable cancer (e.g., MSS colorectal cancer). See, e.g., Vilar and Gruber, Nat Rev Clin Oncol. 2010 Mar;7(3): 153-62.

As used herein, "high levels of repeat RNA" means levels above a reference level or threshold, e.g., a reference that represents a statistically determined threshold, e.g., above which cancer can be diagnosed or treated using a method described herein; conversely, "low levels of repeat RNA" means levels below a reference level or threshold; this can be the same or a different reference level or threshold as is applicable for identifying cancers with high levels of repeat RNA.

In some embodiments, the reference level is the level in a normal cell in the same sample, e.g., a normal cell adjacent to a cancer cell, e.g., a normal adjacent stromal signal. In some embodiments, high levels is defined as a tumor cell signal greater than a normal adjacent stromal cell signal, and low levels are defined as a tumor cell signal that is less than or equal to that seen in the adjacent stromal cells.

Suitable reference levels can be determined by methods known in the art. In some embodiments, the methods include detecting the presence of high levels of repeat RNA, e.g., levels of repeat RNA above a threshold, in a sample from the subject, e.g., a biopsy sample comprising tumor cells or tumor tissue from the subject. Levels of repeat RNA can be determined by any method known in the art, including Northern blot, RNA in situ hybridization (RNA ISH), RNA expression assays, e.g., microarray analysis, RT-PCR, deep sequencing, cloning, Northern blot, and quantitative real time polymerase chain reaction (qRT-PCR) (see International Publication No. WO 2012/048113, which is incorporated by reference herein in its entirety). In some embodiments, in place of detecting high levels of repeat RNA, the methods include detecting copy number of repeat DNA. An increase in copy number as compared to a normal cell, and/or an increase in levels of repeat RNA, indicates that the cancer is susceptible to a treatment described herein. Thus, the methods can include detecting and/or identifying a cancer that has high levels of repeat RNA and/or an increased repeat copy number, and/or selecting a subject who has a cancer with high levels of repeat RNA and/or an increased repeat copy number, for treatment with a method described herein.

In some embodiments, the methods include determining TP53 status of the cancer, and selecting a cancer that harbors a mutation in a TP53 allele (or not selecting a cancer that has wild type TP53). Reference genomic sequences for TP53 can be found at NG_017013.2 (Range 5001 - 24149, RefSeqGene); NC_000017.11 (Range 7668402 - 7687550, Reference GRCh38.p2 Primary Assembly). The methods can include obtaining a sample containing cells from a subject, and evaluating the presence of a mutation in TP53 as known in the art or described herein in the sample, e.g., by comparing the sequence of TP53 in the sample to a reference sequence, e.g., a reference that represents a sequence in a normal (wild-type) or non-cancerous cell, or a disease reference that represents a sequence in a cell from a cancer, e.g., a malignant cell. A mutation in TP53 associated with susceptibility to treatment using a method described herein is a sequence that is different from the reference sequence (e.g., as provided herein) at one or more positions. In some embodiments, the mutation is a mutation known in the art to be associated with cancer. The International Agency for Research on Cancer maintains a database of TP53 mutations found in human cancers, available online at p53.iarc.fr (version R18, April 2016); see also Petitjean et al. (2007) Hum. Mutat. 28(6): 622-9 and Bouaoun et al. (2016) Hum. Mutat. 37(9): 865-76. In some embodiments, the mutation is a mutation at codon 175, 245, 248, 249, 273, or 282. See, e.g., Olivier et al. (2010) Cold Spring Harb. Perspect. Biol. 2(1): a001008.

The presence of a mutation in a TP53, and/or repeat RNA levels and/or repeat copy number, can be evaluated using methods known in the art, *e.g.,* using polymerase chain reaction (PCR), reverse transcriptase polymerase chain reaction (RT-PCR), quantitative or semi-quantitative real-time RT-PCR, digital PCR, *i.e.,* BEAMing (Beads, Emulsion, Amplification, Magnetics), Diehl (2006) Nat Methods 3: 551-559); RNAse protection assay; Northern blot; various types of nucleic acid sequencing (Sanger, pyrosequencing, NextGeneration Sequencing); fluorescent in-situ hybridization (FISH); or gene array/chips); RNA *in situ* hybridization (RNA ISH); RNA expression assays, *e.g.,* microarray analysis; multiplexed gene expression analysis methods, *e.g.,* RT-PCR, RNA-sequencing, and oligo hybridization assays including RNA expression microarrays; hybridization based digital barcode quantification assays such as the nCounter® System (NanoString Technologies, Inc., Seattle, WA; Kulkarni (2011) Curr. Protoc. Mol. Biol. Chapter 25: Unit25B.10), and lysate based hybridization assays utilizing branched DNA signal amplification such as the QuantiGene 2.0 Single Plex and Multiplex Assays (Affymetrix, Inc., Santa Clara, CA; see, e.g., Linton et al. (2012) J. Mol. Diagn. 14(3): 223-32); SAGE, high-throughput sequencing, multiplex PCR, MLPA, Luminex/XMAP, or branched DNA analysis methods. See, *e.g.,* International Publication No. WO 2012/048113, which is incorporated herein by reference in its entirety.

In some embodiments, RNA ISH is used. Certain RNA ISH platforms leverage the ability to amplify the signal within the assay via a branched-chain technique of multiple polynucleotides hybridized to one another (*e.g.,* bDNA) to form a branch structure (*e.g.,* branched nucleic acid signal amplification). In addition to its high sensitivity, the platform also has minimal non-specific background signal compared to immunohistochemistry (see *e.g.,* Urbanek et al. (2015) Int. J. Mol. Sci. 16(6): 13259-86).

In some embodiments, the assay is a bDNA assay as described in U.S. Patent Nos. 7,709,198, 7,803,541, and 8,114,681; and U.S. Publication No. 2006/0263769, which describe the general bDNA approach; see especially 14:39 through 15:19 of the '198 patent. In some embodiments, the methods include using a modified RNA *in situ* hybridization (ISH) technique using a branched-chain DNA assay to directly detect and evaluate the level of biomarker mRNA in the sample (see, *e.g.,* U.S. Patent No. 7,803,541B2; Canales et al. (2006) Nat. Biotechnol. 24(9):1115-22; Ting et al. (2011) Science 331(6017): 593-6). A kit for performing this assay is commercially-available from Affymetrix, Inc. (*e.g.,* the QuantiGene® ViewRNA Assays for tissue and cell samples).

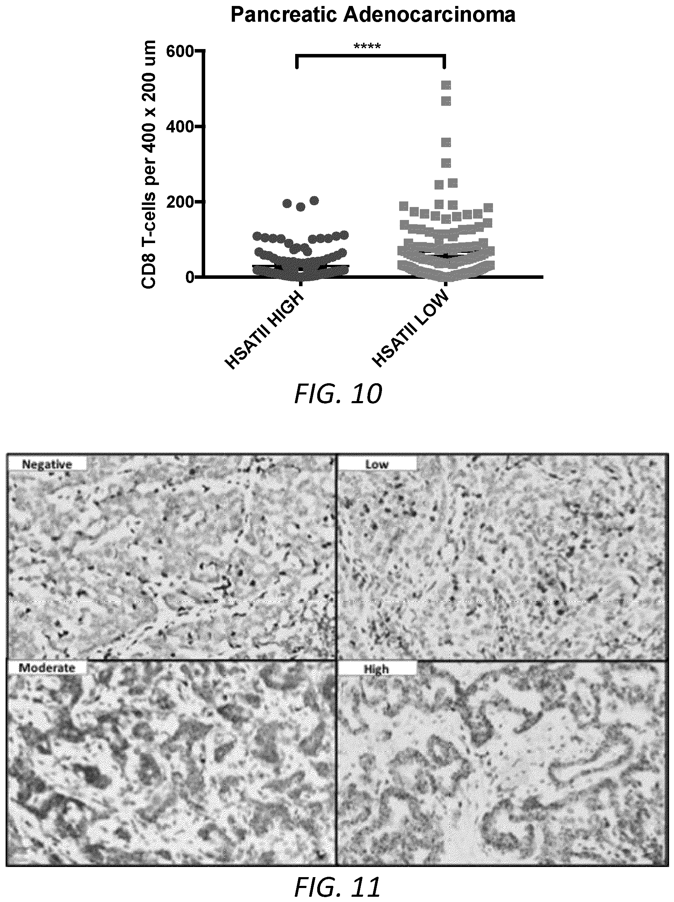

RNA ISH can be performed, *e.g.,* using the QuantiGene® ViewRNA technology (Affymetrix, Santa Clara, CA). QuantiGene® ViewRNA ISH is based on the branched DNA technology wherein signal amplification is achieved via a series of sequential steps (*e.g.,* in a single plex format or a two plex format). Thus, in some embodiments, the methods include performing an assay as described in US Publication No. 2012/0052498 (which describes methods for detecting both a nucleic acid and a protein with bDNA signal amplification, comprising providing a sample comprising or suspected of comprising a target nucleic acid and a target protein; incubating at least two label extender probes each comprising a different L-1 sequence, an antibody specific for the target protein, and at least two label probe systems with the sample comprising or suspected of comprising the target nucleic acid and the target protein, wherein the antibody comprises a pre-amplifier probe, and wherein the at least two label probe systems each comprise a detectably different label; and detecting the detectably different labels in the sample); US Publication No. 2012/0004132; US Publication No. 2012/0003648 (which describes methods of amplifying a nucleic acid detection signal comprising hybridizing one or more label extender probes to a target nucleic acid; hybridizing a pre-amplifier to the one or more label extender probes; hybridizing one or more amplifiers to the pre-amplifier; hybridizing one or more label spoke probes to the one or more amplifiers; and hybridizing one or more label probes to the one or more label spoke probes); or US Publication No. 2012/0172246 (which describes methods of detecting a target nucleic acid sequence, comprising providing a sample comprising or suspected of comprising a target nucleic acid sequence; incubating at least two label extender probes each comprising a different L-1 sequence, and a label probe system with the sample comprising or suspected of comprising the target nucleic acid sequence; and detecting whether the label probe system is associated with the sample). Each hybridized target specific polynucleotide probe acts in turn as a hybridization target for a pre-amplifier polynucleotide that in turn hybridizes with one or more amplifier polynucleotides. In some embodiments, two or more target specific probes (label extenders) are hybridized to the target before the appropriate pre-amplifier polynucleotide is bound to the 2 label extenders, but in other embodiments a single label extender can also be used with a pre-amplifier. Thus, in some embodiments the methods include incubating one or more label extender probes with the sample. In some embodiments, the target specific probes (label extenders) are in a ZZ orientation, cruciform orientation, or other (*e.g.,* mixed) orientation; see, *e.g.,* Figures 10A and 10B of US Publication No. 2012/0052498. Each amplifier molecule provides binding sites to multiple detectable label probe oligonucleotides, *e.g.,* chromogen or fluorophore conjugated-polynucleotides, thereby creating a fully assembled signal amplification "tree" that has numerous binding sites for the label probe; the number of binding sites can vary depending on the tree structure and the labeling approach being used, *e.g.,* from 16-64 binding sites up to 3000-4000 range. In some embodiments there are 300-5000 probe binding sites. The number of binding sites can be optimized to be large enough to provide a strong signal but small enough to avoid issues associated with overlarge structures, *i.e.,* small enough to avoid steric effects and to fairly easily enter the fixed/permeabilized cells and be washed out of them if the target is not present, as larger trees will require larger components that may get stuck within pores of the cells *(e.g.,* the pores created during permeabilization, the pores of the nucleus) despite subsequent washing steps and lead to noise generation.

In some embodiments, the label probe polynucleotides are conjugated to an enzyme capable of interacting with a suitable chromogen, *e.g.,* alkaline phosphatase (AP) or horseradish peroxidase (HRP). Where an alkaline phosphatase (AP)-conjugated polynucleotide probe is used, following sequential addition of an appropriate substrate such as fast red or fast blue substrate, AP breaks down the substrate to form a precipitate that allows in-situ detection of the specific target RNA molecule. Alkaline phosphatase can be used with a number of substrates, *e.g.,* fast red, fast blue, or 5-Bromo-4-chloro-3-indolyl-phosphate (BCIP). Thus, in some embodiments, the methods include the use of alkaline phosphatase conjugated polynucleotide probes within a bDNA signal amplification approach, *e.g.,* as described generally in US Patent Nos. 5,780,277 and 7,033,758. Other enzyme and chromogenic substrate pairs can also be used, *e.g.,* horseradish peroxidase (HRP) and 3,3'-Diaminobenzidine (DAB). Many suitable enzymes and chromogen substrates are known in the art and can be used to provide a variety of colors for the detectable signals generated in the assay, and suitable selection of the enzyme(s) and substrates used can facilitate multiplexing of targets and labels within a single sample. For these embodiments, labeled probes can be detected using known imaging methods, *e.g.,* bright-field microscopy with a CISH approach.

Other embodiments include the use of fluorophore-conjugates probes, *e.g.,* Alexa Fluor dyes (Life Technologies Corporation, Carlsbad, California) conjugated to label probes. In these embodiments, labeled probes can be detected using known imaging methods, *e.g.,* fluorescence microscopy *(e.g.,* FISH). Selection of appropriate fluorophores can also facilitate multiplexing of targets and labels based upon, *e.g.,* the emission spectra of the selected fluorophores.

In some embodiments, the assay is similar to those described in US Publication Nos. 2012/0100540, 2013/0023433, 2013/0171621, 2012/0071343; or 2012/0214152 (the entire contents of each of the foregoing are incorporated herein by reference in their entirety).

In some embodiments, an RNA ISH assay is performed without the use of bDNA, and the repeat RNA (e.g., HSATII, LINE-1, HERV-K, or HERV-H) or TP53 specific probes are directly or indirectly *(e.g.,* via an antibody) labeled with one or more labels as discussed herein.

The assay can be conducted manually or on an automated instrument, such the Leica BOND family of instruments, or the Ventana DISCOVERY ULTRA or DISCOVERY XT instruments.

As used herein, a "test sample" refers to a biological sample obtained from a subject of interest including a cell or cells, *e.g.,* tissue, from a tumor. (Lehninger Biochemistry (Worth Publishers, Inc., current edition); Sambrook et al., Molecular Cloning: A Laboratory Manual, 3d. ed., 2001, Cold Spring Harbor Laboratory Press, New York; Bernard (2002) Clin. Chem. 48(8): 1178-85; Miranda (2010) Kidney International 78: 191-9; Bianchi (2011) EMBO Mol Med 3: 495-503; Taylor (2013) Front. Genet. 4: 142; Yang (2014) PLoS One 9(11): e110641); Nordstrom (2000) Biotechnol. Appl. Biochem. 31(2): 107-12; Ahmadian (2000) Anal. Biochem. 280: 103-10. In some embodiments, high throughput methods, *e.g.,* protein or gene chips as are known in the art (see, *e.g.,* Ch. 12, Genomics, in Griffiths et al., Eds. Modern Genetic Analysis, 1999, W. H. Freeman and Company; Ekins and Chu (1999) Trends in Biotechnology 17: 217-8; MacBeath and Schreiber (2000) Science 289(5485): 1760-3; Simpson, Proteins and Proteomics: A Laboratory Manual, Cold Spring Harbor Laboratory Press; 2002; Hardiman, Microarrays Methods and Applications: Nuts & Bolts, DNA Press, 2003), can be used to detect the presence and/or level of a mutation in TP53.

In some embodiments a technique suitable for the detection of alterations in the structure or sequence of nucleic acids, such as the presence of deletions, amplifications, or substitutions, can be used for the detection of alterations in repeat RNA (e.g., HSATII, LINE-1, HERV-K, or HERV-H) or TP53.

In some embodiments, RT-PCR can be used to detect mutations and copy number variants (CNV). The first step in expression profiling by RT-PCR is the reverse transcription of the RNA template into cDNA, followed by its exponential amplification in a PCR reaction (Ausubel et al. (1997) Current Protocols of Molecular Biology, John Wiley and Sons). To minimize errors and the effects of sample-to-sample variation, RT-PCR is usually performed using an internal standard, which is expressed at constant level among tissues, and is unaffected by the experimental treatment. Housekeeping genes as known in the art are most commonly used.

In some embodiments, the methods can include detecting protein levels of TP53, and comparing the protein levels to reference protein levels in a normal cell. A mutation in TP53 typically results in a decrease in protein expression levels, so a decrease in protein expression levels as compared to a wild type reference or threshold level can be used as a proxy for mutation status; a cancer in which tp53 levels are decreased can be selected for treatment with a method described herein (or a cancer in which TP53 levels are normal or not substantially decreased as compared to a wild type reference or threshold can be excluded from treatment with a method described herein). The level of a protein can be evaluated using methods known in the art, e.g., using standard electrophoretic and quantitative immunoassay methods for proteins, including but not limited to, Western blot; enzyme linked immunosorbent assay (ELISA); biotin/avidin type assays; protein array detection; radio-immunoassay; immunohistochemistry (IHC); immune-precipitation assay; FACS (fluorescent activated cell sorting); mass spectrometry (Kim (2010) Am. J. Clin. Pathol. 134: 157-62; Yasun (2012) Anal. Chem. 84(14): 6008-15; Brody (2010) Expert Rev. Mol. Diagn. 10(8): 1013-22; Philips (2014) PLOS One 9(3): e90226; Pfaffe (2011) Clin. Chem. 57(5): 675-687). The methods typically include detectable labels such as fluorescent, chemiluminescent, radioactive, and enzymatic or dye molecules that provide a signal either directly or indirectly. As used herein, the term "label" refers to the coupling (*i.e.* physically linkage) of a detectable substance, such as a radioactive agent or fluorophore (*e.g.* phycoerythrin (PE) or indocyanine (Cy5), to an antibody or probe, as well as indirect labeling of the probe or antibody (*e.g.* horseradish peroxidase, HRP) by reactivity with a detectable substance.

In some embodiments, an enzyme-linked immunosorbent assay (ELISA) method may be used, wherein the wells of a microtiter plate are coated with an antibody against which the protein is to be tested. The sample containing or suspected of containing the biological marker is then applied to the wells. After a sufficient amount of time, during which antibody-antigen complexes would have formed, the plate is washed to remove any unbound moieties, and a detectably labelled molecule is added. Again, after a sufficient period of incubation, the plate is washed to remove any excess, unbound molecules, and the presence of the labeled molecule is determined using methods known in the art. Variations of the ELISA method, such as the competitive ELISA or competition assay, and sandwich ELISA, may also be used, as these are well-known to those skilled in the art.

In some embodiments, an immunohistochemistry (IHC) method may be used. IHC provides a method of detecting a biological marker *in situ.* The presence and exact cellular location of the biological marker can be detected. Typically a sample (*e.g.,* a biopsy sample) is fixed with formalin or paraformaldehyde, embedded in paraffin, and cut into sections for staining and subsequent inspection by light microscopy. Current methods of IHC typically use either direct or indirect labelling. The sample may also be inspected by fluorescent microscopy when immunofluorescence (IF) is performed, as a variation to IHC.

Mass spectrometry, and particularly matrix-assisted laser desorption/ionization mass spectrometry (MALDI-MS) and surface-enhanced laser desorption/ionization mass spectrometry (SELDI-MS), is useful for the detection of biomarkers of this invention. (See U.S. Patent Nos. 5,118,937; 5,045,694; 5,719,060; and 6,225,047).

The sample can be, *e.g.,* a biopsy, *e.g.,* needle biopsy or a resection specimen, taken from a mass known or suspected to be a tumor or cancerous.

The reference or predetermined level can be a single cut-off (threshold) value, such as a median or mean, or a level that defines the boundaries of an upper or lower quartile, tertile, or other segment of a cohort, *e.g.,* a clinical trial population, that is determined to be statistically different from the other segments. It can be a range of cut-off (or threshold) values, such as a confidence interval. It can be established based upon comparative groups, such as where association with risk of developing disease or presence of disease in one defined group is a fold higher, or lower, (*e.g.,* approximately 2-fold, 4-fold, 8-fold, 16-fold or more) than the risk or presence of disease in another defined group. It can be a range, for example, where a population of subjects (*e.g.,* control subjects) is divided equally (or unequally) into groups, such as a low-risk group, a medium-risk group and a high-risk group, or into quartiles, the lowest quartile being subjects with the lowest risk and the highest quartile being subjects with the highest risk, or into n-quantiles (*i.e.,* n regularly spaced intervals) the lowest of the n-quantiles being subjects with the lowest risk and the highest of the n-quantiles being subjects with the highest risk.

In some embodiments, the amount by which the level in the subject is greater than the reference level is sufficient to distinguish a subject from a control subject, and optionally is statistically significantly greater than the level in a control subject. In cases where the copy number in a subject is "equal to" the reference copy number, the "being equal" refers to being approximately equal (*e.g.,* not statistically different).

The predetermined value can depend upon the particular population of subjects (*e.g.,* human subjects) selected. Appropriate ranges and categories can be selected with no more than routine experimentation by those of ordinary skill in the art.

In characterizing likelihood, or risk, numerous predetermined values can be established.

Selection of an appropriate route of administration of a treatment will depend on various factors including, but not limited to, the particular treatment and/or location of the cancer. In some embodiments, the treatment is administered orally, parenterally, intravenously, topically, intraperitoneally, subcutaneously, intracranially, intrathecally, or by inhalation. In some embodiments, the treatment is administered by continuous infusion.

*Reverse Transcriptase Inhibitors (RTIs)*

Applicants have surprisingly discovered that HSATII HIGH cancer cells that are refractory to treatment with anti-tumor response enhancing immunotherapy become more sensitive when a treatment that reduces immunosuppression is used. In some embodiments, the treatment comprises administration of a reverse transcriptase inhibitor (RTI), e.g., a nucleoside analog reverse transcriptase inhibitor (NRTI), nucleotide analog reverse transcriptase inhibitor, or non-nucleoside reverse transcriptase inhibitor (NNRTI). In some embodiments, the treatment comprises a combination of nucleoside analog reverse transcriptase inhibitors, nucleotide analog reverse transcriptase inhibitors, and/or non-nucleoside reverse transcriptase inhibitors. In some embodiments, the treatment does not include NRTI.

Numerous reverse transcriptase inhibitors are known in the art, and may be used as described herein, including zidovudine (ZDV), didanosine (ddI), stavudine (d4T), zalcitabine (DDC), lamivudine (3TC), abacavir (ABC), tenofovir disoproxil (TDF), emtricitabine (FTC), etravirine lobucavir, entecavir (ETV), apricitabine, censavudine, dexelvucitabine, alovudine, amdoxovir, elvucitabine, racivir, and stampidine. Additional reverse transcriptase inhibitors are disclosed, for example, in U.S. Publication Nos. 2017/0267667, 2016/0145255, 2015/0105351, 2007/0088015, 2013/0296382, 2012/0225894, 2012/0053213, 2012/0029192, 2009/0162319, and 2007/0021442, the entire contents of each of which are incorporated herein by reference. Without wishing to be bound by any particular theory, the use of guanosine and cytidine analogs may be particularly advantageous in the treatment of a cancer comprising high levels of HSATII RNA given that the GC content of HSATII is high. In some embodiments, the RTI is a cytidine analog (e.g., zalcitabine (ddC); lamivudine (3TC); and emtricitabine (FTC). In some embodiments, the RTI is a guanosine analog (e.g., abacavir (ABC), and etecavir (ETV).

## HDAC Inhibitors

In some embodiments, the methods include administration of an HDAC inhibitor, a number of which are known in the art, including Suberoylanilide hydroxamic acid (SAHA/Vorinostat/Zolinza), Trichostatin A (TSA), and PXD-101 (which are hydroxamic acid-based pan-HDAC inhibitors); depsipeptide (FK228/ romidepsin/ISTODAX®) (which is a natural cyclic peptide inhibitor of HDAC1/2); MS-275 and MGCD0103 (which are synthetic benzamide derivatives); and valproic acid and Sodium phenylbutyrate (which are aliphatic acids with relatively low potency). See, e.g., Kim and Bae, Am J Transl Res. 2011 Jan 1; 3(2): 166-179.

## BET Inhibitors

In some embodiments, the methods include administration of a BET inhibitor, a number of which are known in the art, including (+)-JQ1, I-BET762, OTX015, I-BET151, CPI203, PFI-1, MS436, CPI-0610, RVX2135, FT-1101, BAY1238097, INCB054329, TEN-010, GSK2820151, ZEN003694, BAY-299, BMS-986158, ABBV-075, GS-5829, and PLX51107; see, e.g., Pérez-Salvia and Esteller, Epigenetics. 2017; 12(5): 323-339.

## DNA Hypomethylating Agents

In some embodiments, the methods described herein further comprise administering a DNA hypomethylating agent to a subject. DNA methylation is an epigenetic modification that regulates the silencing of gene transcription. Genomic methylation patterns may be altered in tumors (Smet et al. (2010) Epigenetics 5(3): 206-13), and may be of significance in B cell malignancies (Debatin et al. (2007) Cell 129(5): 853-5; Martin-Subero (2006) Leukemia 20(10): 1658-60). As described below, , e.g., treatment with a DNA hypomethylating agent (e.g., 5-azacytidine). Without wishing to be bound by any particular theory, treatment with a DNA hypomethylating agent is believed to activate the transcription of HSATII, which renders the cells susceptible to treatment with a NRTI.

In some embodiments, the DNA hypomethylating agent is a DNA methyltransferase inhibitor. In some embodiments, the DNA methyltransferase inhibitor is 5'-azacytidine (Aza), decitabine (DAC), cladribine (2CdA), or a combination thereof (Wyczechowska et al. (2003) Biochem Pharmacol. 65: 219-25; Yu et al. (2006) Am. J. Hematol. 81(11): 864-9; and Garcia-Manero (2008) Curr. Opin. Oncol. 20(6): 705-10). Additioonal DNA hypomethylating agents are described, for example in U.S. Publication Nos. 2011/0218170A1, 2005/0119201, and 2015/0258068, the entire contents of each of which are incorporated herein by reference.

## HSATII Inhibitory Nucleic Acids

In some embodiments, the methods described herein comprise further administering to a subject an inhibitory nucleic acid (e.g., a LNA molecule) that specifically targets HSATII. Inhibitory nucleic acids targeting HSATII and methods of using the same are disclosed, e.g., in U.S. Publication No. 2017/0198288, the entire contents of which are expressly incorporated herein by reference.

## Immunotherapeutic Agents

In some embodiments, the methods include administering an immunotherapy agent to a subject who is identified using a method described herein. Immunotherapy agents include those therapies that target tumor-induced immune suppression; see, e.g., Stewart and Smyth (2011) Cancer Metastasis Rev. 30(1): 125-40.

## Enhancing the CD8+ T cell Anti-tumor Response

In some embodiments, the present methods including selecting and/or administering a therapy that enhances anti-tumor immune response. Examples of immunotherapies that enhance the CD8+ cell response include, but are not limited to, adoptive T cell therapies or cancer vaccine preparations designed to induce T lymphocytes to recognize cancer cells, as well as checkpoint inhibitors such as anti-CD137 antibodies (e.g., BMS-663513), anti-PD1 antibodies (e.g., Nivolumab, pembrolizumab/MK-3475, Pidilizumab (CT-011)), anti-PDL1 antibodies (e.g., BMS-936559, MPDL3280A), or anti-CTLA-4 antibodies (e.g., ipilumimab; see, e.g., Krüger et al. (2007) Histol Histopathol. 22(6): 687-96; Eggermont et al. (2010) Semin Oncol. 37(5): 455-9; Klinke (2010) Mol. Cancer. 9: 242; Alexandrescu et al. (2010) J. Immunother. 33(6): 570-90; Moschella et al. (2010) Ann N YAcad-Sci. 1194: 169-78; Ganesan and Bakhshi (2010) Natl. Med. J. India 23(1): 21-7; and Golovina and Vonderheide (2010) Cancer J. 16(4): 342-7).

Exemplary anti-PD-1 antibodies that can be used in the methods described herein include those that bind to human PD-1; an exemplary PD-I protein sequence is provided at NCBI Accession No. NP_005009.2. Exemplary antibodies are described in U.S. Patent Nos. 8,008,449; 9,073,994; and U.S. Publication No. 2011/0271358, including, e.g., PF-06801591, AMP-224, BGB-A317, BI 754091, JS001, MEDI0680, PDR001, REGN2810, SHR-1210, TSR-042, pembrolizumab, nivolumab, avelumab, pidilizumab, and atezolizumab.

Exemplary anti-CD40 antibodies that can be used in the methods described herein include those that bind to human CD40; exemplary CD40 protein precursor sequences are provided at NCBI Accession No. NP_001241.1, NP_690593.1, NP_001309351.1, NP_001309350.1 and NP_001289682.1. Exemplary antibodies include those described in International Publication Nos. WO 2002/088186; WO 2007/124299; WO 2011/123489; WO 2012/149356; WO 2012/111762; WO 2014/070934; U.S. Publication Nos. 2013/0011405; 2007/0148163; 2004/0120948; 2003/0165499; and U.S. Patent No. 8,591,900; including, e.g., dacetuzumab, lucatumumab, bleselumab, teneliximab, ADC-1013, CP-870,893, Chi Lob 7/4, HCD122, SGN-4, SEA-CD40, BMS-986004, and APX005M. In some embodiments, the anti-CD40 antibody is a CD40 agonist, and not a CD40 antagonist.

Exemplary anti-PD-L1 antibodies that can be used in the methods described herein include those that bind to human PD-L1; exemplary PD-LI protein sequences are provided at NCBI Accession No. NP_001254635.1, NP_001300958.1, and NP_054862.1. Exemplary antibodies are described in U.S. Publication No. 2017/0058033; International Publication Nos. WO 2016/061142A1; WO 2016/007235A1; WO 2014/195852A1; and WO 2013/079174A1, including, e.g., BMS-936559 (MDX-1105), FAZ053, KN035, Atezolizumab (Tecentriq, MPDL3280A), Avelumab (Bavencio), and Durvalumab (Imfinzi, MEDI-4736).

In some embodiments, immunotherapies can antagonize cell surface receptors to enhance the anti-cancer immune response. For example, antagonistic monoclonal antibodies that boost the anti-cancer immune response can include antibodies that target CTLA-4 (ipilimumab, see Tarhini and Iqbal (2010) Onco Targets Ther. 3: 15-25 and U.S. Patent No. 7,741,345, or tremelimumab) or antibodies that target PD-1 (nivolumab, see Topalian et al. (2012) N. Engl. J. Med. 366(26): 2443-54 and International Publication No. WO 2013/173223, pembrolizumab/MK-3475, and pidilizumab (CT-011)).

Some immunotherapies enhance T cell recruitment to the tumor site (such as endothelin receptor-A/B (ETRA/B) blockade, e.g., with macitentan or the combination of the ETRA and ETRB antagonists BQ123 and BQ788, see Coffman et al. (2013) Cancer Biol Ther. 14(2): 184-92), or enhance CD8 T-cell memory cell formation (e.g., using rapamycin and metformin, see, e.g., Pearce et al. (2009) Nature 460(7251): 103-7; Mineharu et al. (2014) Mol. Cancer Ther. 13(12): 3024-36; and Berezhnoy et al. (2014) Oncoimmunology 3: e28811). Immunotherapies can also include administering one or more of: adoptive cell transfer (ACT) involving transfer of ex vivo expanded autologous or allogeneic tumor-reactive lymphocytes, e.g., dendritic cells or peptides with adjuvant; cancer vaccines such as DNA-based vaccines, cytokines (e.g., IL-2), cyclophosphamide, anti-interleukin-2R immunotoxins, and/or prostaglandin E2 inhibitors (e.g., using SC-50). In some embodiments, the methods include administering a composition comprising tumor-pulsed dendritic cells, e.g., as described in International Publication No. WO 2009/114547 and references cited therein. See also Shiao et al. (2011) Genes & Dev. 25: 2559-72.

*Reducing Tumor-Protective Immunosuppression*

In some embodiments, the present methods including selecting and/or administering a therapy that reduces tumor-protective immunosuppression; these therapies may primarily target immunoregulatory cell types such as regulatory T cells (Tregs) or M2 polarized macrophages, e.g., by reducing number, altering function, or preventing tumor localization of the immunoregulatory cell types. For example, Treg-targeted therapy includes anti-GITR monoclonal antibody (TRX518), cyclophosphamide (e.g., metronomic doses), arsenic trioxide, paclitaxel, sunitinib, oxaliplatin, PLX4720, anthracycline-based chemotherapy, Daclizumab (anti-CD25); immunotoxin e.g., Ontak (denileukin diftitox); lymphoablation (e.g., chemical or radiation lymphoablation) and agents that selectively target the VEGF-VEGFR signalling axis, such as VEGF blocking antibodies (e.g., bevacizumab), or inhibitors of VEGFR tyrosine kinase activity (e.g., lenvatinib) or ATP hydrolysis (e.g., using ectonucleotidase inhibitors, e.g., ARL67156 (6-N,N-Diethyl-D-$\beta,\gamma$-dibromomethyleneATP trisodium salt), 8-(4-chlorophenylthio) cAMP (pCPT-cAMP) and a related cyclic nucleotide analog (8-[4-chlorophenylthio] cGMP; pCPT-cGMP) and those described in International Publication No. WO 2007/135195, as well as monoclonal antibodies (mAbs) against CD73 or CD39). Docetaxel also has effects on M2 macrophages. See, e.g., Zitvogel et al. (2013) Immunity 39: 74-88.

In another example, M2 macrophage targeted therapy includes clodronate-liposomes (Zeisberger, et al. (2006) Br. J. Cancer 95: 272-81), DNA based vaccines (Luo et al. (2006) J. Clin. Invest. 116(8): 2132-41), and M2 macrophage targeted pro-apoptotic peptides (Cieslewicz et al. (2013) Proc. Natl. Acad. Sci. U.S.A. 110(40): 15919-24). Macrophages can also be targeted using CSF-1R inhibitors (e.g., PLX3397, AMG820 IMC-CS4/LY3022855, or RG7155/RO5509554) or other small molecules or blocking monoclonal antibodies (e.g., against CD11b (e.g., Rovelizumab); CCL2 (e.g., Carlumab); ANG2 (e.g., Nesvacumab); IL4 (e.g., Pascolizumab); IL4-Ra (e.g., Dupilumab); IL13 (e.g., Lebrikizumab, Tralokinumab, GSK679586); FcgammaR (e.g., Rituximab (CD20); Ibrutinib (BTK); R788 (Syk)); Neuropilin-1 (e.g., MNRP1685A); IL-6 (e.g., Clazakizumab, Olokizumab, Siltuximab, Sirukumab, IL-6R (e.g., Tocilizumab, Sarilumab); TNF-$\alpha$ (e.g., MAPK inhibitors, e.g., Adalimumab, Certolizumab, Etanercept, Golimumab, Infliximab); or CD40 (e.g., CP-870,893). See, e.g., Ruffell and Coussens, Cancer Cell. 2015 Apr 13; 27(4): 462-472 and references cited therein.

Some useful immunotherapies target the metabolic processes of immunity, and include adenosine receptor antagonists and small molecule inhibitors, e.g., istradefylline (KW-6002) and SCH-58261; indoleamine 2,3-dioxygenase (IDO) inhibitors, e.g., small molecule inhibitors (e.g., 1-methyl-tryptophan (1MT), 1-methyl-d-tryptophan (D1MT), and Toho-1) or IDO-specific siRNAs, or natural products (e.g., brassinin or exiguamine) (see, e.g., Munn (2012) Front. Biosci. (Elite Ed).4: 734-45) or monoclonal antibodies that neutralize the metabolites of IDO, e.g., mAbs against N-formyl-kynurenine.

In some embodiments, the immunotherapies may antagonize the action of cytokines and chemokines such as IL-10, TGF-$\beta$, IL-6, CCL2 and others that are associated with immunosuppression in cancer. For example, TGF-$\beta$ neutralizing therapies include anti-TGF-$\beta$ antibodies (e.g. fresolimumab, infliximab, lerdelimumab, GC-1008), antisense oligodeoxynucleotides (e.g., trabedersen), and small molecule inhibitors of TGF-beta (e.g. LY2157299) (Wojtowicz-Praga (2003) Invest. New Drugs 21(1): 21-32). Another example of therapies that antagonize immunosuppression cytokines can include anti-IL-6 antibodies (e.g. siltuximab) (Guo, et al., Cancer Treat Rev. 38(7):904-910 (2012). mAbs against IL-10 or its receptor can also be used, e.g., humanized versions of those described in Llorente et al., Arthritis & Rheumatism, 43(8): 1790-1800, 2000 (anti-IL-10 mAb), or Newton et al., Clin Exp Immunol. 2014 Jul;177(1):261-8 (anti-interleukin-10R1 monoclonal antibody). mAbs against CCL2 or its receptors can also be used. In some embodiments, the cytokine immunotherapy is combined with a commonly used cytotoxic chemotherapeutic agent (e.g., gemcitabine, docetaxel, cisplatin, tamoxifen), e.g., as described in U.S. Patent No. 8,476,246.

In some embodiments, immunotherapies can include agents that are believed to elicit "danger" signals, e.g., "PAMPs" (pathogen-associated molecular patterns) or "DAMPs" (damage-associated molecular patterns) that stimulate an immune response against the cancer. See, e.g., Pradeu and Cooper (2012) Front Immunol. 3: 287; Escamilla-Tilch et al. (2013) Immunol. Cell. Biol. 91(10): 601-10. In some embodiments, immunotherapies can agonize toll-like receptors (TLRs) to stimulate an immune response. For example, TLR agonists include vaccine adjuvants (e.g., 3M-052) and small molecules (e.g., imiquimod, muramyl dipeptide, CpG, and mifamurtide (muramyl tripeptide)), as well as polysaccharide krestin and endotoxin. See, Galluzi et al. (2012) Oncoimmunol. 1(5): 699-716, Lu et al. (2011) Clin. Cancer Res. 17(1): 67-76, and U.S. Patent Nos. 8,795,678 and 8,790,655. In some embodiments, immunotherapies can involve administration of cytokines that elicit an anti-cancer immune response, see Lee & Margolin (2011) Cancers 3: 3856-93. For example, the cytokine IL-12 can be administered (Portielje, et al. (2003) Cancer Immunol. Immunother. 52: 133-44) or as gene therapy (Melero et al. (2001) Trends Immunol. 22(3): 113-5). In another example, interferons (IFNs), e.g., IFNgamma, can be administered as adjuvant therapy (Dunn et al. (2006) Nat. Rev. Immunol. 6: 836-48).

*Pharmaceutical Compositions*

The methods described herein can include the administration of pharmaceutical compositions and formulations comprising a therapeutic agent described herein.

In some embodiments, the compositions are formulated with a pharmaceutically acceptable carrier. The pharmaceutical compositions and formulations can be administered parenterally, topically, orally or by local administration, such as by aerosol or transdermally. The pharmaceutical compositions can be formulated in any way and can be administered in a variety of unit dosage forms depending upon the condition or disease and the degree of illness, the general medical condition of each subject, the resulting preferred method of administration and the like. Details on techniques for formulation and administration of pharmaceuticals are well described in the scientific and patent literature, see, e.g., Remington: The Science and Practice of Pharmacy, 21st ed., 2005.

The therapeutic agent can be administered as a single active agent in a pharmaceutical composition or in combination with other active agents. The compositions may be formulated for administration, in any convenient way for use in human or veterinary medicine. Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Formulations of the compositions include those suitable for intradermal, inhalation, oral/ nasal, topical, parenteral, rectal, and/or intravaginal administration. The formulations may conveniently be presented in unit dosage form and may be prepared by any methods well known in the art of pharmacy. The amount of active ingredient (*e.g.,* a therapeutic agent) that can be combined with a carrier material to produce a single dosage form will vary depending upon the host being treated, the particular mode of administration, *e.g.,* intradermal, parenteral, intravenous, or via inhalation. The amount of active ingredient which can be combined with a carrier material to produce a single dosage form will generally be that amount of the compound which produces a therapeutic effect.

Pharmaceutical formulations of this invention can be prepared according to any method known to the art for the manufacture of pharmaceuticals, and can contain sweetening agents, flavoring agents, coloring agents and preserving agents. A formulation can be admixtured with nontoxic pharmaceutically acceptable excipients which are suitable for manufacture. Formulations may comprise one or more diluents, emulsifiers, preservatives, buffers, excipients, etc. and may be provided in such forms as liquids, powders, emulsions, lyophilized powders, sprays, creams, lotions, controlled release formulations, tablets, pills, gels, on patches, in implants, etc.

Pharmaceutical formulations for oral administration can be formulated using pharmaceutically acceptable carriers well known in the art in appropriate and suitable dosages. Such carriers enable the pharmaceuticals to be formulated in unit dosage forms as tablets, pills, powder, dragees, capsules, liquids, lozenges, gels, syrups, slurries, suspensions, etc., suitable for ingestion by the subject. Pharmaceutical preparations for oral use can be formulated as a solid excipient, optionally grinding a resulting mixture, and processing the mixture of granules, after adding suitable additional compounds, if desired, to obtain tablets or dragee cores. Suitable solid excipients are carbohydrate or protein fillers include, *e.g.,* sugars, including lactose, sucrose, mannitol, or sorbitol; starch from corn, wheat, rice, potato, or other plants; cellulose such as methyl cellulose, hydroxypropylmethylcellulose, or sodium carboxy-methylcellulose; and gums including arabic and tragacanth; and proteins, *e.g.,* gelatin and collagen. Disintegrating or solubilizing agents may be added, such as the cross-linked polyvinyl pyrrolidone, agar, alginic acid, or a salt thereof, such as sodium alginate. Push-fit capsules can contain active agents mixed with a filler or binders such as lactose or starches, lubricants such as talc or magnesium stearate, and, optionally, stabilizers. In soft capsules, the active agents can be dissolved or suspended in suitable liquids, such as fatty oils, liquid paraffin, or liquid polyethylene glycol with or without stabilizers.

Aqueous suspensions can contain an active agent (*e.g.,* nucleic acid sequences of the invention) in admixture with excipients suitable for the manufacture of aqueous suspensions, *e.g.,* for aqueous intradermal injections. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (*e.g.,* lecithin), a condensation product of an alkylene oxide with a fatty acid (*e.g.,* polyoxyethylene stearate), a condensation product of ethylene oxide with a long chain aliphatic alcohol (*e.g.,* heptadecaethylene oxycetanol), a condensation product of ethylene oxide with a partial ester derived from a fatty acid and a hexitol (*e.g.,* polyoxyethylene sorbitol mono-oleate), or a condensation product of ethylene oxide with a partial ester derived from fatty acid and a hexitol anhydride (*e.g.,* polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

In some embodiments, oil-based pharmaceuticals are used for administration of a therapeutic blocking agent described herein. Oil-based suspensions can be formulated by suspending an active agent in a vegetable oil, such as arachis oil, olive oil, sesame oil or coconut oil, or in a mineral oil such as liquid paraffin; or a mixture of these. See *e.g.,* U.S. Patent No. 5,716,928 describing using essential oils or essential oil components for increasing bioavailability and reducing inter- and intra-individual variability of orally administered hydrophobic pharmaceutical compounds (see also U.S. Patent No. 5,858,401). The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto (1997) J. Pharmacol. Exp. Ther. 281:93-102.

Pharmaceutical formulations can also be in the form of oil-in-water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent. In alternative embodiments, these injectable oil-in-water emulsions of the invention comprise a paraffin oil, a sorbitan monooleate, an ethoxylated sorbitan monooleate and/or an ethoxylated sorbitan trioleate.

The pharmaceutical compounds can also be administered by in intranasal, intraocular and intravaginal routes including suppositories, insufflation, powders and aerosol formulations (for examples of steroid inhalants, see *e.g.,* Rohatagi (1995) J. Clin. Pharmacol. 35:1187-1193; Tjwa (1995) Ann. Allergy Asthma Immunol. 75:107-111). Suppositories formulations can be prepared by mixing the drug with a suitable nonirritating excipient which is solid at ordinary temperatures but liquid at body temperatures and will therefore melt in the body to release the drug. Such materials are cocoa butter and polyethylene glycols.

In some embodiments, the pharmaceutical compounds can be delivered transdermally, by a topical route, formulated as applicator sticks, solutions, suspensions, emulsions, gels, creams, ointments, pastes, jellies, paints, powders, and aerosols.

In some embodiments, the pharmaceutical compounds can also be delivered as microspheres for slow release in the body. For example, microspheres can be administered via intradermal injection of drug which slowly release subcutaneously; see Rao (1995) J. Biomater Sci. Polym. Ed. 7:623-645; as biodegradable and injectable gel formulations, see, *e.g.,* Gao (1995) Pharm. Res. 12:857-863 (1995); or, as microspheres for oral administration, see, *e.g.,* Eyles (1997) J. Pharm. Pharmacol. 49:669-674.

In some embodiments, the pharmaceutical compounds can be parenterally administered, such as by intravenous (IV) administration or administration into a body cavity or lumen of an organ. These formulations can comprise a solution of active agent dissolved in a pharmaceutically acceptable carrier. Acceptable vehicles and solvents that can be employed are water and Ringer's solution, an isotonic sodium chloride. In addition, sterile fixed oils can be employed as a solvent or suspending medium. For this purpose any bland fixed oil can be employed including synthetic mono- or diglycerides. In addition, fatty acids such as oleic acid can likewise be used in the preparation of injectables. These solutions are sterile and generally free of undesirable matter. These formulations may be sterilized by conventional, well known sterilization techniques. The formulations may contain pharmaceutically acceptable auxiliary substances as required to approximate physiological conditions such as pH adjusting and buffering agents, toxicity adjusting agents, *e.g.,* sodium acetate, sodium chloride, potassium chloride, calcium chloride, sodium lactate and the like. The concentration of active agent in these formulations can vary widely, and will be selected primarily based on fluid volumes, viscosities, body weight, and the like, in accordance with the particular mode of administration selected and the subject's needs. For IV administration, the formulation can be a sterile injectable preparation, such as a sterile injectable aqueous or oleaginous suspension. This suspension can be formulated using those suitable dispersing or wetting agents and suspending agents. The sterile injectable preparation can also be a suspension in a nontoxic parenterally-acceptable diluent or solvent, such as a solution of 1,3-butanediol. The administration can be by bolus or continuous infusion (*e.g.,* substantially uninterrupted introduction into a blood vessel for a specified period of time).

In some embodiments, the pharmaceutical compounds and formulations can be lyophilized. Stable lyophilized formulations comprising an oligo can be made by lyophilizing a solution comprising a pharmaceutical of the invention and a bulking agent, e.g., mannitol, trehalose, raffinose, and sucrose or mixtures thereof. A process for preparing a stable lyophilized formulation can include lyophilizing a solution about 2.5 mg/mL protein, about 15 mg/mL sucrose, about 19 mg/mL NaCl, and a sodium citrate buffer having a pH greater than 5.5 but less than 6.5. See, *e.g.,* U.S. 20040028670.

The compositions and formulations can be delivered by the use of liposomes. By using liposomes, particularly where the liposome surface carries ligands specific for target cells, or are otherwise preferentially directed to a specific organ, one can focus the delivery of the active agent into target cells *in vivo*. See, *e.g.,* U.S. Patent Nos. 6,063,400; 6,007,839; Al-Muhammed (1996) J. Microencapsul. 13:293-306; Chonn (1995) Curr. Opin. Biotechnol. 6:698-708; Ostro (1989) Am. J. Hosp. Pharm. 46:1576-1587. As used in the present invention, the term "liposome" means a vesicle composed of amphiphilic lipids arranged in a bilayer or bilayers. Liposomes are unilamellar or multilamellar vesicles that have a membrane formed from a lipophilic material and an aqueous interior that contains the composition to be delivered. Cationic liposomes are positively charged liposomes that are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes can also include "sterically stabilized" liposomes, i.e., liposomes comprising one or more specialized lipids. When incorporated into liposomes, these specialized lipids result in liposomes with enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Pat. No. 6,287,860.

The formulations of the invention can be administered for prophylactic and/or therapeutic treatments. In some embodiments, for therapeutic applications, compositions are administered to a subject who is at risk of or has a disorder described herein, in an amount sufficient to cure, alleviate or partially arrest the clinical manifestations of the disorder or its complications; this can be called a therapeutically effective amount.

The amount of pharmaceutical composition adequate to accomplish this is a therapeutically effective dose. The dosage schedule and amounts effective for this use, *i.e.,* the dosing regimen, will depend upon a variety of factors, including the stage of the disease or condition, the severity of the disease or condition, the general state of the subject's health, the subject's physical status, age and the like. In calculating the dosage regimen for a subject, the mode of administration also is taken into consideration.

The dosage regimen also takes into consideration pharmacokinetics parameters well known in the art, *i.e.,* the active agents' rate of absorption, bioavailability, metabolism, clearance, and the like (see, *e.g.,* Hidalgo-Aragones (1996) J. Steroid Biochem. Mol. Biol. 58:611-617; Groning (1996) Pharmazie 51:337-341; Fotherby (1996) Contraception 54:59-69; Johnson (1995) J. Pharm. Sci. 84:1144-1146; Rohatagi (1995) Pharmazie 50:610-613; Brophy (1983) Eur. J. Clin. Pharmacol. 24:103-108; Remington: The Science and Practice of Pharmacy, 21st ed., 2005). The state of the art allows the clinician to determine the dosage regimen for each individual subject, active agent and disease or condition treated. Guidelines provided for similar compositions used as pharmaceuticals can be used as guidance to determine the dosage regimen, *i.e.,* dose schedule and dosage levels, administered practicing the methods of the invention are correct and appropriate.

Single or multiple administrations of formulations can be given depending on for example: the dosage and frequency as required and tolerated by the subject, the degree and amount of therapeutic effect generated after each administration (*e.g.,* effect on tumor size or growth), and the like. The formulations should provide a sufficient quantity of active agent to effectively treat, prevent or ameliorate conditions, diseases or symptoms (*e.g.,* cancer).

## EXAMPLES

The invention is further described in the following examples, which do not limit the scope of the invention described in the claims.

## Example 1

All combined RNA-ISH and IHC assays are performed as follows: Monoclonal antibodies for immune cells (T-cell subsets CD3, CD4, CD8, FOXP3; Macrophages CD68, CD163; B-cells CD20; NK cells CD16, CD56) and RNA ISH probes for repeat RNAs (HSATII, HERV-K, HERV-H, LINE1) were sequentially applied on a single histologic slide, and contrasting chromogens were used to visualize the antibody: brown (diaminobenzidine [DAB]) for immune marker and RNA: red (fast red) for repeat RNA. Paraffin-embedded sections were mounted on coated slides and were placed in an oven for 60 minutes at 60°C. The sequential double-staining protocol was performed using the Leica Bond Rx automated immunostainer. Deparaffinization (View RNA Dewax1 protocol) and onboard antigen retrieval were performed for 20 minutes at approximately 100°C with HIER2 reagent, which is an EDTA based proprietary Leica solution (pH 8.0-8.5). Monoclonal antibodies for each immune cell subset was diluted per protocol in Leica antibody diluent solution. For ISH part View- RNA eZL Detection Kit (Affymetrix) was used on the Bond RX immunohistochemistry and ISH Staining System with BDZ 6.0 software (Leica Biosystems). The Bond RX user-selectable settings for part 2 were as follows: ViewRNA eZ-l Detection 1-plex (Red) protocol; ViewRNA Dewax1 Preparation protocol; ViewRNA Enzyme 2 (10); ViewRNA Probe Hybridization 3hrs , With these settings, the RNA unmasking conditions for the FFPE tissue consisted: 10-minute incubation with Proteinase K from the Bond Enzyme Pretreatment Kit at 1:1000 dilution (Leica Biosystems). RNA repeat Ez probes were diluted as 1:40 in ViewRNA Probe Diluent (Affymetrix). Post run, slides were rinsed with water, air dried for 30 minutes at room temperature and mounted using Dako Ultramount (Dako, Carpinteria,CA), and visualized using a standard brightfield microscope. Punctate dot like red color hybridization signals in the cell cytoplasm and nucleus defined as positive signals for repeat RNA and brown cytoplasmic reactivity in lymphocytes was considered as positive for immune marker.

## Example 2

The immunotherapy field has seen that CD8+ T cell infiltrates in general correlate to response to immunotherapy (CTLA4 or PD1/PDL1) (Taube JM et al. Mod Pathol. 2017 Dec 1. doi: 10. 1 038/modpathol.2017. 156). FOXP3+ regulatory T cells are thought to block the anti-tumor T cell response though this data has been more conflicted (see, e.g., Manjili and Butler, Immunol Invest. 2016 Nov;45(8):759-766; Ward-Hartstonge and Kemp, Clin Transl Immunology. 2017 Sep 15;6(9):e154).

We predicted that HSATII low tumors would correlate to HIGH CD8 T cell infiltrates and HERV-H high tumors would correlate to LOW FOXP3+ cells, which would be most responsive to immunotherapy.

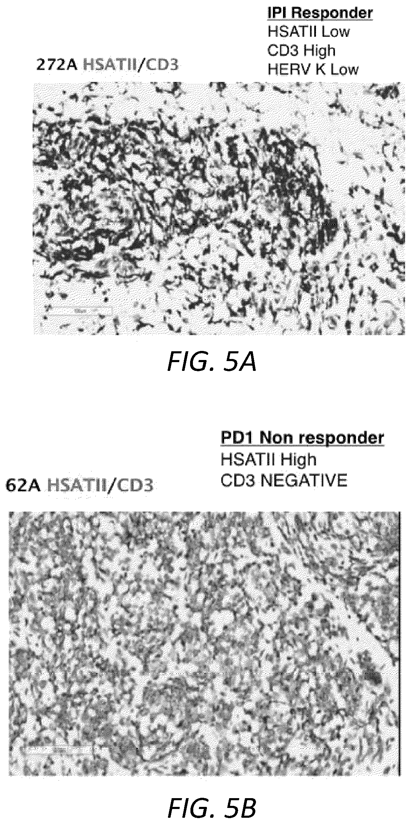

As shown herein, HSATII low melanomas respond to immunotherapy including anti-PD1/PDL1 and anti-CTLA4 therapies (Figure 5). Briefly, a cohort of 20 melanoma patients who received either anti-PD1/PDL1 or anti-CTLA4 therapy that had formalin fixed paraffin embedded (FFPE) tissue were evaluated with HSATII RNA-ISH combined with CD3 T cell IHC assay. Tumors were evaluated for HSATII RNA levels (HIGH vs LOW) and CD3 T cell infiltrates quantitated by manual counting of cells in a $400 \times 200$ micron area by a pathologist. The CD3 T cell infiltrates were significantly lower in HSATII HIGH vs LOW tumors (p < 0.02). There was a relationship of HSATII expression and immunotherapy response as determined by standard clinical imaging criteria (CT scan change), where HSATII LOW tumors were responsive and HSATII HIGH tumors not responsive to these checkpoint immunotherapy drugs.

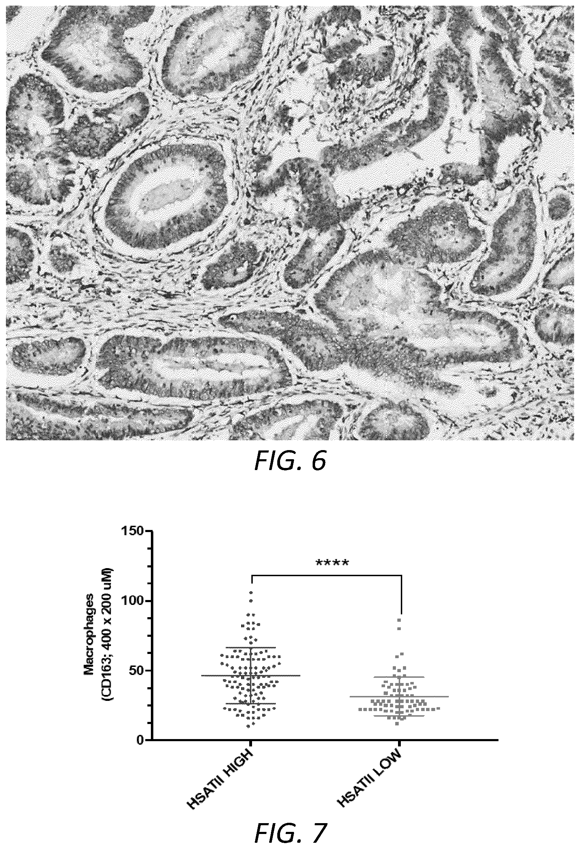

In addition, the present data indicates that HSATII high signal in a cohort of human colon cancer is positively correlated with CD163+ tumor associated macrophages (Figures 6 & 7), which is typically associated with an immunosuppressive or non-responsive tumor microenvironment (Ruffelll and Coussens, Cancer Cell. 2015 Apr 13; 27(4): 462-472). Briefly, primary colon cancers on a tissue microarray were stained with HSATII RNA-ISH combined with CD163 macrophage IHC assay. Tumors were evaluated for HSATII RNA levels (HIGH vs LOW) and CD163 macrophage infiltrates quantitated by manual counting of cells in a $400 \times 200$ micron area by a pathologist. CD163 macrophages were significantly higher in HSATII HIGH colon cancers (**** $p < 0.0001$).

**Example 3**

HSATII and CD163+ macrophages were positively correlated in pancreatic ductal adenocarcinoma (PDAC). HSATII high expression was associated with higher CD163+ macrophages. Therefore, HSATII in tumor cells is a surrogate marker of macrophage infiltrates which can be used to select patients for drugs targeting macrophages in the tumor immune microenvironment. This finding was consistent with the data for colon cancer in Figures 6 and 7.

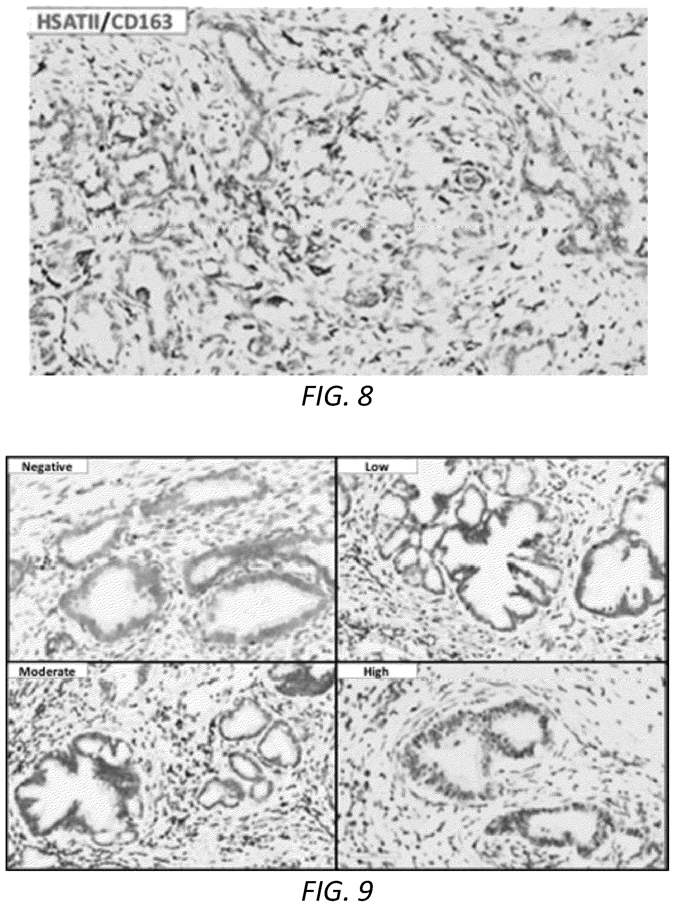

Quantitation of CD8+ T-cells by IHC in PDAC tumors separated by HSATII HIGH (high, moderate) vs LOW (low, negative) expression are shown (Figure 8).

**Example 4**

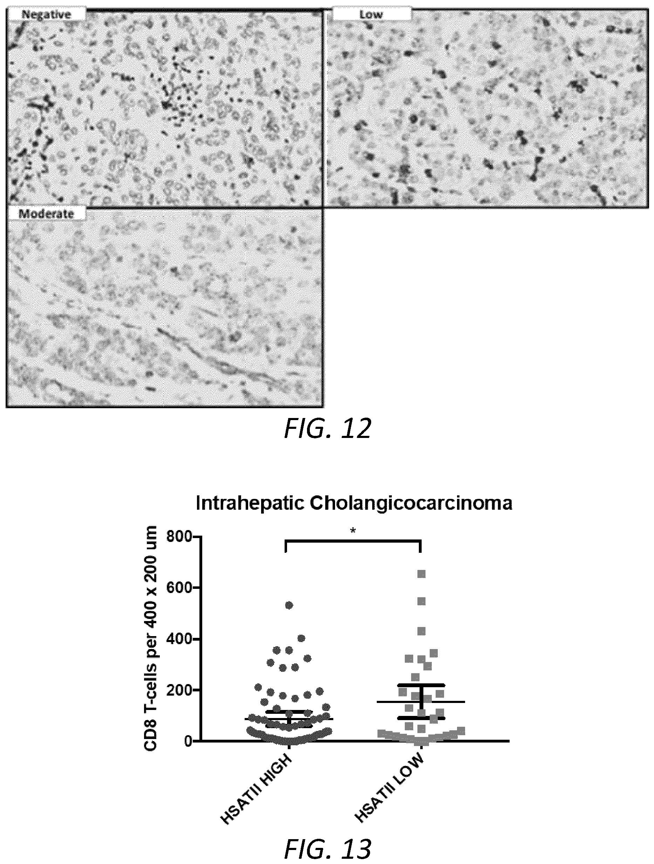

HSATII and CD8+ T-cells were negatively correlated in pancreatic ductal adenocarcinoma (Figure 9), intrahepatic cholangiocarcinoma (Figure 11), and hepatocellular carcinoma (Figure 12) demonstrating utility of this marker as a surrogate of cytolytic immune cell infiltrates, which would be useful in determining tumors that are amenable to immune checkpoint inhibition (anti-CTLA4 and/or anti-PD1/PDL1). This extend on the data in colon cancer published (Solovyov A et al. Cell Reports 2018).

Quantitation of CD8 T-cells was performed in PDAC (Figure 10) and ICC (Figure. 13) tumors separated by HSATII HIGH (high, moderate) vs LOW (low, negative) expression.

**Example 5**

Experimental Procedures

We selected 38 samples from TCGA which had total RNA frozen solid tumor RNA-Seq data. These samples were comprised of 12 LUAD, 10 COAD, 5 BRCA, 4 KIRC, 4 UCEC, 3 BLCA tumors. Out of these 38 samples, 29 samples had matching poly(A) RNA-Seq data. Total RNA and poly(A) prepared aliquots were derived from the same physical sample. These samples were comprised of 11 LUAD, 6 COAD, 5 BRCA, 4 KIRC, 3 BLCA tumors. The presence of such paired samples allowed one to perform a technical comparison of sequencing protocols and their effects on computed gene expression.

The total RNA and the poly(A) preparation protocols used different strategies for the ribosomal RNA (rRNA) depletion. The total RNA protocol employed RiboZero kit to remove rRNA. The poly(A) used the poly(A) capture procedure to isolate the polyadenylated transcripts which leaves rRNA out. After initial quality filtering we aligned the reads to the human genome and to the Repbase database of repetitive elements (Bao et al. 2015). The number of reads mapping to the annotated genomic features was quantified and expression was computed.

Gene expression in terms of log2-CPM (counts per million reads) was computed and normalized across samples using the TMM method as implemented in the calcNormFactors function of edgeR package (Robinson et. al., 2010). Only coding genes were used for normalization. In particular, this procedure assures that the computational subtraction of the rRNA reads is done. The purpose of the normalization procedure was to identify some reference quantities (e.g., housekeeping gene expression) which can be compared between the different samples to establish the sample specific normalization factor. In particular, the TMM normalization procedure assumed that most of the genes were not differentially expressed or that the effects of the overexpression and the underexpression were approximately equal except for some outliers. These assumptions were reasonable when we consider the protocol specific difference for the coding genes. Indeed, the majority of the coding genes were expected to be detectable by both protocols, which was not the case for the repeat elements. Genes with low expression (ones not having at least 10 reads per million reads in at least two samples) were filtered out. The same protocol was used for all datasets.

The difference of the computed expression between the two protocols was computed using limma package (Smyth, 2004; Ritchie et. al., 2015). Expression data were used in conjuction with the weights computed by the voom transformation (Law et. al., 2014). Despite the use of the same computational procedure (paired t-test as implemented in limma package), this "differential expression" test measured the technical difference between the two sequencing protocols, not the biological difference between the various tissues. This difference was expressed as the binary logarithm of the fold change (logFC).

Chi squared test for the variance of computed gene expression was performed as follows. We considered only genes with median expression using both poly(A) and total RNA protocol exceeded log2(10). For every physical sample we computed the difference between the expression values from poly(A) protocol and total RNA protocol. Then we computed the variance of these differences. We performed the chi-squared test for the variance to verify whether these differences are sample independent. We required that the linear fold change between the two biological conditions (e.g., tumor and normal tissue) FC=2 is detectable, we assuming n=3 replicates for each of the conditions. This led to the cutoff for the variance used in the test. Adjusted p-values (FDR) were computed using Benjamini & Hochberg method.

We performed the linear regression between the variance and the log of the repeat length in the genome. For the rank correlation rho we have performed the linear regression between log((1+rho)/(1-rho)) and the log of the repeat length in the genome (logistic regression).

Clustering of repeat elements based on expression was performed as follows. We have created 1000 bootstrap datasets and performed clustering on each of them. After that we computed the consensus clustering. Entropic forces acting on the sequence motifs were computed using the methods developed in (Greenbaum et.al., 2012).

Gene ontology enrichment analyses were performed using the web tools powered by PANTHER (Thomas et. al., 2003; Mi et. al., 2009). We used "GO biological process complete" annotation set (GO ontology database retrieved 2017-01-26) with PANTHER overrepresentation test (release 20160715) with Bonferroni correction.

GSEA analysis was performed using the pre-ranked gene list. Genes in the list were ranked according to the t-statistic from the differential expression analysis. The list included all expressed genes, not necessarily differentially expressed genes. We acknowledge our use of the gene set enrichment analysis, GSEA software, and Molecular Signature Database (MsigDB), http://www.broad.mit.edu/gsea/ (Subramanian et. al., 2005).

Results

ERV class expression can be associated with positive anti-PD-L1 immunotherapy response.

Pre-existing tumor T cell inflammation can be a strong predictor of response to cancer immunotherapy such as anti-PD-L1/PD-1 or anti-CTLA-4 antibodies (Chen et al. Nature 2017, 541:321-330). Several studies have recently highlighted links between tumors ERV expression, the expression of "viral defense genes", and anti-tumor responses (Chiapinelli et al. Cell 2015, 162, 974-986; Roulois et al. Cell 2015, 162:961-973; Badal et al. JCI Insight 2017, 2, e92102). We hypnotize that chemically-induced epigenetic dysregulation in tumors leads to expression of ERVs, which in turn stimulate innate immune PRRs and create an anti-tumoral innate immune response. In one of these studies (Chiapinelli et al. 2015), endogenous ERV presence was associated with clinical benefit in patients treated with anti-CTLA-4 therapy. We examined one of the few available tumor immunotherapy RNA-seq datasets from patients treated with PD-L1 blockade (Snyder et al. 2017). In this cohort of patients with urothelial cancer, we tested the hypothesis that ERV expression is also associated with clinical benefit from therapy. We performed this analysis for the first time in an anti-PDL1 treated tumor, as opposed to the previous anti-CTLA4 studies.

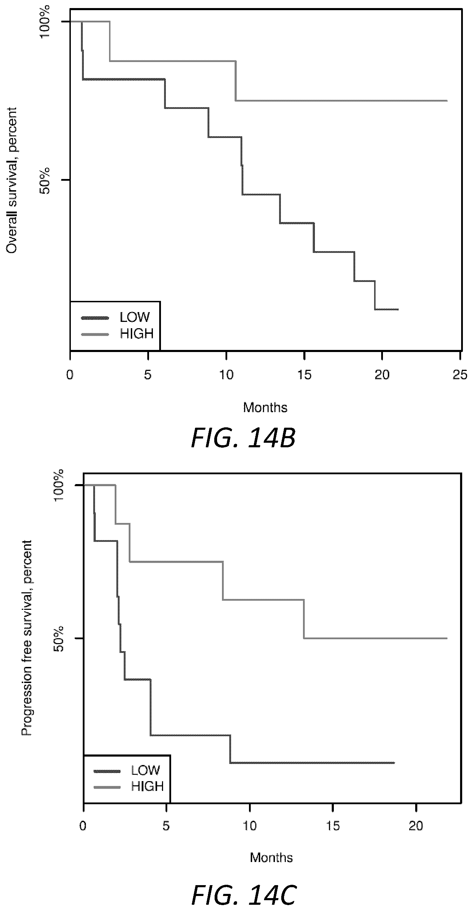

We performed hierarchical clustering using expression of ERV repeats using the repeatmasker/Repbase annotation, which revealed two distinct clusters of high and low ERV expression levels. In this case, association between ERV repeats expression and patient response to PD-L1 immunotherapy was significant (p=0.024, Fisher's exact test). Consequently, patient survival analysis showed that high expression of ERV repeats correlates with overall survival (Figure. 14B, p=0.012) and progression free survival (Figure. 14C, p=0.025). We performed logistic regression for the clinical benefit vs. the total ERV repeat expression,

$$\log \frac{p}{1-p} = -7.0 + 2.4 E_{ERV};$$

where E_ERV is the total expression of ERV repeats and p is the probability of a clinical benefit. The coefficient for E_ERV is significant (p-value = 0.04).

We performed Cox regression for the overall survival (*hazard* = -2.9 * $E_{ERV}$ + 0.4 * *age* + 3.2 * *met; where $E_{ERV}$* is the total expression of ERV repeats, age is the patients' age and met is 1 when liver metastases are present and 0 otherwise). Coefficients for $E_{ERV}$ and met are significant (p=0.001 and p=0.003). We performed the Cox regression for the progression free survival (*hazard* = -1.5 * $E_{ERV}$ - 1.9 * *age* + 1.8 * met). Coefficients for $E_{ERV}$ and met are significant (p=0.009 and p=0.02). In both cases we performed a test for the proportional hazards assumption, and the assumption holds.

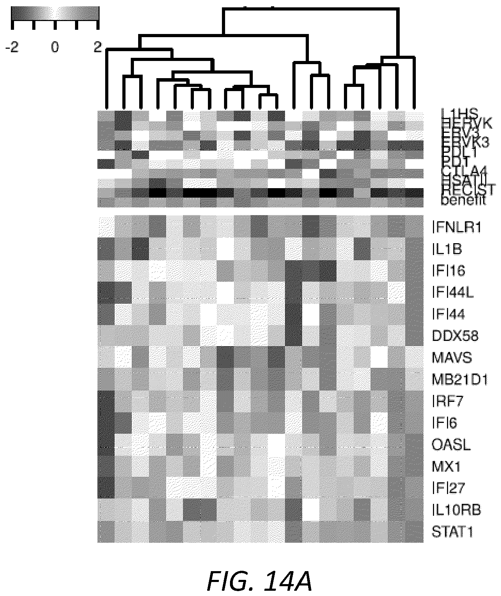

Interestingly, expression of ERV repeats was a better predictor of response to immunotherapy than the viral defense signature in this cohort, which did not similarly segregate patients (Figure 14A). We performed a series of Cox regressions for the hazard ratio using the patient's age, presence of liver metastases and expression of one of the viral defense genes or ERVs as independent variables. The effects of ERV expression were associated with improved survival and statistically significant (p=0.001, FDR=0.02). Effects of the viral defense genes were not statistically significant. Additionally, as we show that Repeatmasker/Repbase annotation for ERV repeats yields a higher read number than that for ERV genes annotated in Ensembl, we suggest that clinical studies would reveal more accurate associations by interrogating global repeat expression for a particular class of repeats rather than specific ERV genes annotated in Ensembl or their associated immune classes. It is worth mentioning that the read counts of the ERV genes annotated in Ensembl were below the standard 10 reads per million threshold in RNA-Seq, ERV3 and ERV3K having the highest read number. Expression of these two genes was correlated with the mean ERV expression. The implication is that, due to the abundant transcription of repetitive elements, they are a more robust predictor of response to immunotherapy than the expression of associated immune genes, which likely require a larger sample size to resolve cohorts.

In addition, we investigated the dataset of (Hugo et.al.) and performed a similar series of Cox regressions using age, number of non-synonymous mutations and expression of one of the viral defense genes or ERVs as independent variables. Neither expression of the viral defense genes nor that of ERVs had a statistically significant effect on the hazard ratio. It is worth noting that almost all the tumors in this dataset are metastatic unlike the dataset of (Snyder et. al.). Likewise this dataset was for melanoma, which may have different patterns of ERV expression than urothelial cancer that may make their presence more difficult to assess. Altogether, this suggests that there are unique repeat classes linked with different phenotypes that are tissue context dependent, which merits further investigation.

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**The invention will now be defined by the following clauses**

1. A method of treating a subject with cancer in a subject, the method comprising:

providing a sample comprising cells from the cancer;
detecting a level of repeat RNA (e.g., HSATII, LINE-1, HERV-K, HERV-H) in the cells;
comparing the level of repeat RNA in the sample to a reference level; and
selecting and optionally administering a treatment comprising one or both of (i) a treatment that reduces levels of export of repeat RNA or increases levels of repeat RNA in the cells, or (ii) a tumor-protective immunosuppression reducing immunotherapy, to a subject who has a cancer that has levels of repeat RNA above a reference level.

2. The method of clause 1, wherein the treatment that reduces levels of export of repeat RNA or increases levels of repeat RNA in the cells is an inhibitory nucleic acid targeting repeat RNA, preferably selected from the group consisting of a locked nucleic acid (LNA) molecule, a short hairpin RNA (shRNA) molecule, a small inhibitory RNA (siRNA) molecule, an antisense nucleic acid molecule, a peptide nucleic acid molecule, and a morpholino.

3. The method of clause 1, wherein the treatment that levels of export of repeat RNA or increases levels of repeat RNA in the cells is a reverse transcriptase inhibitor (RTI) selected from the group consisting of a nucleoside analog reverse transcriptase inhibitor, a nucleotide analog reverse transcriptase inhibitor, non-nucleoside reverse transcriptase inhibitor, and a combination thereof.

4. The method of clause 3, wherein the nucleoside analog reverse transcriptase inhibitor comprises lamivudine, abacavir, zidovudine, emtricitabine, didanosine, stavudine, entecavir, apricitabine, censavudine, zalcitabine, dexelvucitabine, amdoxovir, elvucitabine, festinavir, racivir, stampidine, or a combination thereof.

5. The method of clause 3, wherein the non-nucleoside reverse transcriptase inhibitor comprises lersivirine, rilpivirine, efavirenz, etravirine, doravirine, dapivirine, or a combination thereof; or wherein the nucleotide analog reverse transcriptase inhibitor comprises tenofovir alafenamide fumarate, tenofovir disoproxil fumarate, adefovir, or a combination thereof.

6. The method of clause 1, wherein the treatment that reduces levels of export of repeat RNA or increases levels of repeat RNA in the cells comprises a cytidine analog or a guanosine analog.

7. The method of clause 1, wherein the treatment that reduces levels of export of repeat RNA or increases levels of repeat RNA in the cells comprises an HDAC inhibitor; a BET inhibitor; or a DNA hypomethylating agent.

8. The method of clause 7, wherein the DNA hypomethylating agent is azacytidine, decitabine, cladribine, or a combination thereof; the HDAC inhibitor is Suberoylanilide hydroxamic acid (SAHA/Vorinostat/Zolinza), Trichostatin A (TSA), PXD-101, depsipeptide, MS-275, MGCD0103, valproic acid, or Sodium phenylbutyrate, or the BET inhibitor is (+)-JQ1, I-BET762, OTX015, I-BET151, CPI203, PFI-1, MS436, CPI-0610, RVX2135, FT-1101, BAY1238097, INCB054329, TEN-010, GSK2820151, ZEN003694, BAY-299, BMS-986158, ABBV-075, GS-5829, or PLX51107.

9. The method of clause 1, comprising administering the treatment comprising one or both of (i) a treatment that reduces levels of export of repeat RNA or increases levels of repeat RNA in the cells or (ii) a tumor-protective immunosuppression reducing immunotherapy, for a time sufficient to reduce levels of repeat below a subsequent reference level, and then administering a treatment comprising an anti-tumor immunity enhancing immunotherapy to the subject, wherein the initial and subsequent reference levels can be the same or different.

10. The method of clause 9, wherein the anti-tumor immunity enhancing immunotherapy comprises an immunotherapy agent selected from the group consisting of an anti-PD-1 antibody, an anti-PD-L1 antibody, an anti-CD137 antibody, an anti-CTLA4 antibody, an anti-CD40 antibody, an anti-IL10 antibody, an anti-TGF-β antibody, and an anti-IL-6 antibody.

11. The method of any one of clauses 1-10, wherein the administering the treatment results in a reduction in tumor burden in the subject.

12. The method of any one of clauses 1-11, wherein the cancer is an epithelial cancer.

13. The method of clause 12, wherein the epithelial cancer is melanoma, pancreatic cancer, colorectal cancer, breast cancer, prostate cancer, renal cancer, ovarian cancer, lung cancer, pancreatic cancer, liver cancer, or urothelial cancer.

14. The method of any one of clauses 1-13, wherein the cancer comprises a mutation in tumor protein p53 (TP53).

15. A method of predicting whether a cancer will respond to a treatment comprising an anti-tumor immunity enhancing immunotherapy, the method comprising:

providing a sample comprising cells from the cancer;
detecting a level of repeat RNA in the cells; and comparing the level of repeat RNA in the sample to a reference level; wherein a cancer that has levels of repeat RNA below the reference level is likely to respond to a treatment comprising an anti-tumor immunity enhancing immunotherapy.

16. A method of predicting whether a cancer will respond to a treatment comprising a tumor-protective immunosuppression reducing immunotherapy, the method comprising:

providing a sample comprising cells from the cancer;
detecting a level of repeat RNA in the cells; and comparing the level of repeat RNA in the sample to a reference level; wherein a cancer that has levels of repeat RNA above the reference level is likely to respond to a treatment comprising a tumor-protective immunosuppression reducing immunotherapy, an inhibitory nucleic acid targeting repeat RNA, or a reverse transcriptase inhibitor (RTI) selected from the group consisting of a nucleoside analog reverse transcriptase inhibitor, a nucleotide analog reverse transcriptase inhibitor, non-nucleoside reverse transcriptase inhibitor, and a combination thereof.

17. A method of determining a level of immune cells in a cancer, the method comprising:

providing a sample comprising cells from the cancer;
detecting a level of HSATII and/or HERV-H repeat RNA in the cells; and
comparing the level of HSATII and/or HERV-H repeat RNA in the sample to a reference level (e.g., a level in a normal adjacent cell or tissue); wherein:

a cancer that has levels of HSATII repeat RNA above the reference level has CD8 T-cells in the cancer;
a cancer that has levels of HSATII repeat RNA at or below the reference level has CD163 macrophages; and
a cancer that has levels of HERV-H repeat RNA above the reference level has FOXP3 T cells.

18. The method of clause 17, further comprising categorizing the cancer as CD8+, CD163+, or FOXP3+ based on the level of HSATII and/or HERV-H in the cells.

19. The method of clause 13, wherein the epithelial cancer is pancreatic ductal adenocarcinoma, intrahepatic cholangiocarcinoma, or hepatocellular carcinoma.

**Claims**

1. One or both of (i) a treatment that reduces levels of export of repeat RNA or increases levels of repeat RNA in the cells, or (ii) a tumor-protective immunosuppression reducing immunotherapy for use in a method of treating a subject with cancer in a subject, after the cancer has been identified by

providing a sample comprising cells from the cancer;

detecting a level of repeat RNA (e.g., HSATII, LINE-1, HERV-K, HERV-H) in the cells; comparing the level of repeat RNA in the sample to a reference level; wherein the method comprises selecting and administering said treatment comprising one or both of (i) a treatment that reduces levels of export of repeat RNA or increases levels of repeat RNA in the cells, or (ii) a tumor-protective immunosuppression reducing immunotherapy, to the subject who has the cancer having the levels of repeat RNA if the levels of repeat RNA are above the reference level.

2. The treatment of (i) and/or immunotherapy of (ii) for the use according to claim 1, wherein the repeat RNA is HSATII; wherein (i) and (ii) further comprise (iii) an anti-tumor immunity enhancing immunotherapy; wherein:

after one or both of the treatment of (i) and the immunotherapy of (ii), has been administered to the subject for a time sufficient to reduce levels of HSATII below a subsequent reference level, then the treatment comprising (iii) the anti-tumor immunity enhancing immunotherapy administered to the subject, wherein the initial and subsequent reference levels can be the same or different,

wherein (i), the treatment that reduces levels of export of HSATII or increases levels of HSATII in cells, comprises a reverse transcriptase inhibitor (RTI) selected from the group consisting of a nucleoside analog reverse transcriptase inhibitor, a nucleotide analog reverse transcriptase inhibitor, non-nucleoside reverse transcriptase inhibitor, and a combination thereof,

(ii), the tumor-protective immunosuppression reducing immunotherapy, is selected from the group consisting of: an anti-GITR monoclonal antibody, cyclophosphamide, arsenic trioxide, paclitaxel, sunitinib, oxaliplatin, PLX4720, anthracycline-based chemotherapy, an anti-CD25 antibody; denileukin diftitox; lymphoablation, bevacizumab, Lenvatinib, ARL67156 (6-N,N-Diethyl -D-P,y-dibromom ethylene ATP trisodium salt), 8-(4-chlorophenylthio) cAMP (pCPT-cAMP) and a related cyclic nucleotide analog (8-[4-chlorophenylthio] cGMP; pCPT-cGMP), clodronate-liposomes, DNA based vaccines, M2 macrophage targeted pro-apoptotic peptides, a CSF-1R inhibitor, an anti-CD11b antibody, an anti-CCL2 antibody, an anti-ANG2 antibody, an anti-IL4 antibody, an anti-IL4-Ra antibody, an anti-IL13 antibody, an anti-CD20 antibody, an IL-6R antibody, an anti-TNF-α antibody, an anti-CD40 antibody, istradefylline, SCH-58261, an indoleamine 2,3-dioxygenase (IDO) inhibitor, brassinin, exiguamine, an anti-N-formyl-kynurenine antibody, an antagonist of IL-10, TGF-β, IL-6, or CCL2, fresolimumab, infliximab, lerdelimumab, GC-1008, trabedersen, LY2157299, siltuximab, an anti-CCL2 antibody, gemcitabine, docetaxel, cisplatin, tamoxifen, a TLR agonists, and IFNgamma, and

(iii), the anti-tumor immunity enhancing immunotherapy, is an anti-PD1 antibody or an anti-CTLA4 antibody.

3. The treatment of (i) and/or immunotherapy of (ii) for the use according to claim 1, wherein the treatment of (i) further comprises an inhibitory nucleic acid targeting repeat RNA, preferably selected from the group consisting of a locked nucleic acid (LNA) molecule, a short hairpin RNA (shRNA) molecule, a small inhibitory RNA (siRNA) molecule, an antisense nucleic acid molecule, a peptide nucleic acid molecule, and a morpholino.

4. The treatment of (i) and/or immunotherapy of (ii) for the use according to claim 1, wherein the nucleoside analog reverse transcriptase inhibitor comprises lamivudine, abacavir, zidovudine, emtricitabine, didanosine, stavudine, entecavir, apricitabine, censavudine, zalcitabine, dexelvucitabine, amdoxovir, elvucitabine, festinavir, racivir, stampidine, or a combination thereof; or the non-nucleoside reverse transcriptase inhibitor comprises lersivirine, rilpivirine, efavirenz, etravirine, doravirine, dapivirine, or a combination thereof; or wherein the nucleotide analog reverse transcriptase inhibitor comprises tenofovir alafenamide fumarate, tenofovir disoproxil fumarate, adefovir, or a combination thereof.

5. The treatment of (i) and/or immunotherapy of (ii) for the use according to any one of claims 1 to 4, wherein the treatment of (i) comprises a cytidine analog or a guanosine analog.

6. The treatment of (i) and/or immunotherapy of (ii) for the use according to any one of claims 1 to 4, wherein the treatment of (i) comprises an HDAC inhibitor; a BET inhibitor; or a DNA hypomethylating agent.

7. The treatment of (i) and/or immunotherapy of (ii) for the use according to claim 6, wherein the DNA hypomethylating agent is azacytidine, decitabine, cladribine, or a combination thereof; the HDAC inhibitor is Suberoylanilide hydroxamic acid (SAHA/Vorinostat/Zolinza), Trichostatin A (TSA), PXD-101, depsipeptide, MS-275, MGCD0103, valproic acid, or Sodium phenylbutyrate, or the BET inhibitor is (+)-JQ1, I-BET762, OTX015, I-BET151, CPI203, PFI-1, MS436, CPI-0610, RVX2135, FT-1101, BAY1238097, INCB054329, TEN-010, GSK2820151, ZEN003694, BAY-299, BMS-986158, ABBV-075, GS-5829, or PLX51107.

8. The treatment of (i) and/or immunotherapy of (ii) for the use according to any one of claims 1-6, wherein the administering the treatment results in a reduction in tumor burden in the subject.

9. The treatment of (i) and/or immunotherapy of (ii) for the use according to of any one of claims 1-7, wherein the cancer is an epithelial cancer; and optionally the epithelial cancer is melanoma, pancreatic cancer, colorectal cancer, breast cancer, prostate cancer, renal cancer, ovarian cancer, lung cancer, pancreatic cancer, liver cancer, or urothelial cancer.

10. The treatment of (i) and/or immunotherapy of (ii) for the use according to any one of claims 1-8, wherein the cancer comprises a mutation in tumor protein p53 (TP53).

11. The treatment of (i) and/or immunotherapy of (ii) for the use according to any one of claims 2-10, wherein the cancer is categorized as CD8+, CD163+, or FOXP3+ based on the level of HSATII.

12. A method of predicting whether a cancer will respond to a treatment comprising an anti-tumor immunity enhancing immunotherapy, the method comprising:

  providing a sample comprising cells from the cancer;
  detecting a level of repeat RNA in the cells; and comparing the level of repeat RNA in the sample to a reference level; wherein a cancer that has levels of repeat RNA below the reference level is likely to respond to a treatment comprising an anti-tumor immunity enhancing immunotherapy.

13. A method according to claim 12, wherein the repeat RNA is HSATII.

HERVH / Treg(FOXP3+)

*FIG. 1A*

HERVH / Treg(FOXP3+)

*FIG. 1B*

*FIG. 2*

Low HSATII / High CD8+ c lls

*FIG. 3A*

High HSATII / Low CD8+ cells

*FIG. 3B*

*FIG. 4*

**IPI Responder**
HSATII Low
CD3 High
272A HSATII/CD3    HERV K Low

## FIG. 5A

**PD1 Non responder**
HSATII High
62A HSATII/CD3    CD3 NEGATIVE

## FIG. 5B

FIG. 6

FIG. 7

*FIG. 8*

*FIG. 9*

FIG. 10

FIG. 11

FIG. 12

**Intrahepatic Cholangicocarcinoma**

FIG. 13

*FIG. 14A*

*FIG. 14B*

*FIG. 14C*

30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62627151
- WO 2012048113L A
- US 20170198288 A1
- WO 2012048113 A
- US 7709198 B
- US 7803541 B
- US 8114681 B
- US 20060263769 A
- US 7803541 B2
- US 20120052498 A
- US 20120004132 A
- US 20120003648 A
- US 20120172246 A
- US 5780277 A
- US 7033758 B
- US 20120100540 A
- US 20130023433 A
- US 20130171621 A
- US 20120071343 A
- US 20120214152 A
- US 5118937 A
- US 5045694 A
- US 5719060 A
- US 6225047 B
- US 20170267667 A
- US 20160145255 A
- US 20150105351 A
- US 20070088015 A
- US 20130296382 A
- US 20120225894 A
- US 20120053213 A
- US 20120029192 A
- US 20090162319 A
- US 20070021442 A
- US 20110218170 A1
- US 20050119201 A1
- US 20150258068 A1
- US 20170198288 A
- US 8008449 B
- US 9073994 B
- US 20110271358 A
- WO 2002088186 A
- WO 2007124299 A
- WO 2011123489 A
- WO 2012149356 A
- WO 2012111762 A
- WO 2014070934 A
- US 20130011405 A
- US 20070148163 A
- US 20040120948 A
- US 20030165499 A
- US 8591900 B
- US 20170058033 A
- WO 2016061142 A1
- WO 2016007235 A1
- WO 2014195852 A1
- WO 2013079174 A1
- US 7741345 B
- WO 2013173223 A
- WO 2009114547 A
- WO 2007135195 A
- US 8476246 B
- US 8795678 B
- US 8790655 B
- US 5716928 A
- US 5858401 A
- US 20040028670 A
- US 6063400 A
- US 6007839 A
- US 6287860 B

**Non-patent literature cited in the description**

- **PROSSER et al.** *J. Mol. Biol.,* 1986, vol. 187 (2), 145-55
- **WARBURTON et al.** *BMC Genomics,* 2008, vol. 9, 533
- **TING et al.** *Science,* 2011, vol. 331 (6017), 593-6
- **SNYDER et al.** *PLoS Med.,* vol. 14, e1002309
- **ROONEY et al.** *Cell,* 2015, vol. 160, 48-61
- **CHIAPPINELLI et al.** *Cell,* 2015, vol. 162, 974-986
- **ROULOIS et al.** *Cell,* 2015, vol. 162, 961-973
- **LEONOVA et al.** *Proc Natl Acad Sci U S A,* 2013, vol. 110, 89-98
- **TANNE et al.** *Proc Natl Acad Sci U S A,* 2015, vol. 112, 15154-15159
- **DESAI et al.** *JCI Insight,* 2017, vol. 2, e91078
- **TING et al.** *Science,* 2011, vol. 331, 593-596
- **SOLOVYOV et al.** *bioRxiv,* 2017

- **MANJILI MH ; BUTLER SE.** *Immunological Investigators,* 2016
- **WARD-HARTSTONGE KA ; KEMP RA.** *Clinical & Translational Immunology,* 2017
- **BERSANI et al.** *Proc. Natl. Acad. Sci. U.S.A.,* 2015, vol. 112 (49), 15148-53
- **SHARMA.** *N. Engl. J. Med.,* 2009, vol. 361 (26), 2548-56
- *N Engl J Med.,* vol. 362 (15), 1450
- **VILAR ; GRUBER.** *Nat Rev Clin Oncol.,* March 2010, vol. 7 (3), 153-62
- **PETITJEAN et al.** *Hum. Mutat.,* 2007, vol. 28 (6), 622-9
- **BOUAOUN et al.** *Hum. Mutat.,* 2016, vol. 37 (9), 865-76
- **OLIVIER et al.** *Cold Spring Harb. Perspect. Biol.,* 2010, vol. 2 (1), a001008
- **DIEHL.** *Nat Methods,* 2006, vol. 3, 551-559
- **KULKARNI.** Curr. Protoc. Mol. Biol. NanoString Technologies, Inc, 2011
- **LINTON et al.** J. Mol. Diagn. Affymetrix, Inc, 2012, vol. 14, 223-32
- **URBANEK et al.** *Int. J. Mol. Sci.,* 2015, vol. 16 (6), 13259-86
- **CANALES et al.** *Nat. Biotechnol.,* 2006, vol. 24 (9), 1115-22
- **LEHNINGER.** Biochemistry. Worth Publishers, Inc,
- **SAMBROOK et al.** Molecular Cloning: A Laboratory Manual. Cold Spring Harbor Laboratory Press, 2001
- **BERNARD.** *Clin. Chem.,* 2002, vol. 48 (8), 1178-85
- **MIRANDA.** *Kidney International,* 2010, vol. 78, 191-9
- **BIANCHI.** *EMBO Mol Med,* 2011, vol. 3, 495-503
- **TAYLOR.** *Front. Genet.,* 2013, vol. 4, 142
- **YANG.** *PLoS One,* 2014, vol. 9 (11), e110641
- **NORDSTROM.** *Biotechnol. Appl. Biochem.,* 2000, vol. 31 (2), 107-12
- **AHMADIAN.** *Anal. Biochem.,* 2000, vol. 280
- Genomics. Modern Genetic Analysis. W. H. Freeman and Company, 1999
- **EKINS ; CHU.** *Trends in Biotechnology,* 1999, vol. 17, 217-8
- **MACBEATH ; SCHREIBER.** *Science,* 2000, vol. 289 (5485), 1760-3
- **SIMPSON.** Proteins and Proteomics: A Laboratory Manual. Cold Spring Harbor Laboratory Press, 2002
- **HARDIMAN.** Microarrays Methods and Applications: Nuts & Bolts. DNA Press, 2003
- **AUSUBEL et al.** Current Protocols of Molecular Biology. John Wiley and Sons, 1997
- **KIM.** *Am. J. Clin. Pathol.,* 2010, vol. 134, 157-62
- **YASUN.** *Anal. Chem.,* 2012, vol. 84 (14), 6008-15
- **BRODY.** *Expert Rev. Mol. Diagn.,* 2010, vol. 10 (8), 1013-22
- **PHILIPS.** *PLOS One,* 2014, vol. 9 (3), e90226
- **PFAFFE.** *Clin. Chem.,* 2011, vol. 57 (5), 675-687
- **KIM ; BAE.** *Am J Transl Res.,* 01 January 2011, vol. 3 (2), 166-179
- **PÉREZ-SALVIA ; ESTELLER.** *Epigenetics,* 2017, vol. 12 (5), 323-339
- **SMET et al.** *Epigenetics,* 2010, vol. 5 (3), 206-13
- **DEBATIN et al.** *Cell,* 2007, vol. 129 (5), 853-5
- **MARTIN-SUBERO.** *Leukemia,* 2006, vol. 20 (10), 1658-60
- **WYCZECHOWSKA et al.** *Biochem Pharmacol.,* 2003, vol. 65, 219-25
- **YU et al.** *Am. J. Hematol.,* 2006, vol. 81 (11), 864-9
- **GARCIA-MANERO.** *Curr. Opin. Oncol.,* 2008, vol. 20 (6), 705-10
- **STEWART ; SMYTH.** *Cancer Metastasis Rev.,* 2011, vol. 30 (1), 125-40
- **KRÜGER et al.** *Histol Histopathol.,* 2007, vol. 22 (6), 687-96
- **EGGERMONT et al.** *Semin Oncol.,* 2010, vol. 37 (5), 455-9
- **KLINKE.** *Mol. Cancer.,* 2010, vol. 9, 242
- **ALEXANDRESCU et al.** *J. Immunother.,* 2010, vol. 33 (6), 570-90
- **MOSCHELLA et al.** *Ann N Y Acad Sci.,* 2010, vol. 1194, 169-78
- **GANESAN ; BAKHSHI.** *Natl. Med. J. India,* 2010, vol. 23 (1), 21-7
- **GOLOVINA ; VONDERHEIDE.** *Cancer J.,* 2010, vol. 16 (4), 342-7
- **TARHINI ; IQBAL.** *Onco Targets Ther.,* 2010, vol. 3, 15-25
- **TOPALIAN et al.** *N. Engl. J. Med.,* 2012, vol. 366 (26), 2443-54
- **COFFMAN et al.** *Cancer Biol Ther.,* 2013, vol. 14 (2), 184-92
- **PEARCE et al.** *Nature,* 2009, vol. 460 (7251), 103-7
- **MINEHARU et al.** *Mol. Cancer Ther.,* 2014, vol. 13 (12), 3024-36
- **BEREZHNOY et al.** *Oncoimmunology,* 2014, vol. 3, e28811
- **SHIAO et al.** *Genes & Dev.,* 2011, vol. 25, 2559-72
- **ZITVOGEL et al.** *Immunity,* 2013, vol. 39, 74-88
- **ZEISBERGER et al.** *Br. J. Cancer,* 2006, vol. 95, 272-81
- **LUO et al.** *J. Clin. Invest.,* 2006, vol. 116 (8), 2132-41

- **CIESLEWICZ et al.** *Proc. Natl. Acad. Sci. U.S.A.,* 2013, vol. 110 (40), 15919-24
- **RUFFELL ; COUSSENS.** *Cancer Cell.,* 13 April 2015, vol. 27 (4), 462-472
- **MUNN.** Front. Biosci. 2012, vol. 4, 734-45
- **WOJTOWICZ-PRAGA.** *Invest. New Drugs,* 2003, vol. 21 (1), 21-32
- **GUO et al.** *Cancer Treat Rev.,* 2012, vol. 38 (7), 904-910
- **LLORENTE et al.** *Arthritis & Rheumatism,* 2000, vol. 43 (8), 1790-1800
- **NEWTON et al.** *Clin Exp Immunol.,* vol. 177 (1), 261-8
- **PRADEU ; COOPER.** *Front Immunol.,* 2012, vol. 3, 287
- **ESCAMILLA-TILCH et al.** *Immunol. Cell. Biol.,* 2013, vol. 91 (10), 601-10
- **GALLUZI et al.** *Oncoimmunol.,* 2012, vol. 1 (5), 699-716
- **LU et al.** *Clin. Cancer Res.,* 2011, vol. 17 (1), 67-76
- **LEE ; MARGOLIN.** *Cancers,* 2011, vol. 3, 3856-93
- **PORTIELJE et al.** *Cancer Immunol. Immunother.,* 2003, vol. 52, 133-44
- **MELERO et al.** *Trends Immunol.,* 2001, vol. 22 (3), 113-5
- **DUNN et al.** *Nat. Rev. Immunol.,* 2006, vol. 6, 836-48
- Remington: The Science and Practice of Pharmacy. 2005
- **MINTO.** *J. Pharmacol. Exp. Ther.,* 1997, vol. 281, 93-102
- **ROHATAGI.** *J. Clin. Pharmacol.,* 1995, vol. 35, 1187-1193
- **TJWA.** *Ann. Allergy Asthma Immunol.,* 1995, vol. 75, 107-111
- **RAO.** *J. Biomater Sci. Polym. Ed.,* 1995, vol. 7, 623-645
- **GAO.** *Pharm. Res.,* 1995, vol. 12, 857-863
- **EYLES.** *J. Pharm. Pharmacol.,* 1997, vol. 49, 669-674
- **AL-MUHAMMED.** *J. Microencapsul.,* 1996, vol. 13, 293-306
- **CHONN.** *Curr. Opin. Biotechnol.,* 1995, vol. 6, 698-708
- **OSTRO.** *Am. J. Hosp. Pharm.,* 1989, vol. 46
- **HIDALGO-ARAGONES.** *J. Steroid Biochem. Mol. Biol.,* 1996, vol. 58, 611-617
- **GRONING.** *Pharmazie,* 1996, vol. 51, 337-341
- **FOTHERBY.** *Contraception,* 1996, vol. 54, 59-69
- **JOHNSON.** *J. Pharm. Sci.,* 1995, vol. 84, 1144-1146
- **ROHATAGI.** *Pharmazie,* 1995, vol. 50, 610-613
- **BROPHY.** *Eur. J. Clin. Pharmacol.,* 1983, vol. 24, 103-108
- **TAUBE JM et al.** *Mod Pathol.,* 01 December 2017
- **MANJILI ; BUTLER.** *Immunol Invest.,* November 2016, vol. 45 (8), 759-766
- **WARD-HARTSTONGE ; KEMP.** *Clin Transl Immunology.,* 15 September 2017, vol. 6 (9), e154
- **RUFFELLL ; COUSSENS.** *Cancer Cell.,* 13 April 2015, vol. 27 (4), 462-472
- **SOLOVYOV A et al.** *Cell Reports,* 2018
- **CHEN et al.** *Nature,* 2017, vol. 541, 321-330
- **CHIAPINELLI et al.** *Cell,* 2015, vol. 162, 974-986
- **BADAL et al.** *JCI Insight,* 2017, vol. 2, e92102